(19) Europäisches Patentamt
European Patent Office
Office européen des brevets

(11) EP 1 890 152 A1

(12) EUROPEAN PATENT APPLICATION

(43) Date of publication:
**20.02.2008 Bulletin 2008/08**

(51) Int Cl.:
***G01N 33/68*** (2006.01) ***C07K 14/72*** (2006.01)
***G01N 33/74*** (2006.01) ***C12Q 1/34*** (2006.01)

(21) Application number: **06016940.6**

(22) Date of filing: **14.08.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Charite Universitätsmedizin-Berlin**
**10117 Berlin (DE)**

(72) Inventors:
- **Funke-Kaiser, Heiko, Dr. med.**
  **10825 Berlin (DE)**
- **Schefe, Jan H.**
  **10559 Berlin (DE)**
- **Unger, Thomas, Prof. Dr. Med.**
  **14195 Berlin (DE)**
- **Zollmann, Frank S., Dr. med.**
  **12161 Berlin (DE)**

(74) Representative: **Engelhard, Markus**
**Forrester & Boehmert**
**Pettenkoferstrasse 20-22**
**80336 München (DE)**

# (54) Determination of renin/prorenin receptor activity

(57) The present invention refers to the renin/prorenin receptor (RER) signal transduction pathway and, in particular, to the role of promyelocytic zinc finger protein (PLZF) and its downstream targets involved in this pathway, e.g. the p85α subunit of phosphatidylinositol-3 kinase (PI3K-p85α). In more detail, the present invention refers to a method for determination of RER activity, e.g. stimulation or inhibition of RER activity, using PLZF activity as a measurement from which RER activity is derived. For the determination of RER activity, use can be made of RER/PLZF protein interaction, PLZF translocation and/or PLZF recruitment. The present invention further refers to a use of said method for identifying RER ligands, e.g. pharmaceutically active agonists or antagonists, as well as for studying undesired side-effects of renin inhibitors.

EP 1 890 152 A1

SEQUENCE LISTING

<110> Charité - Universitätsmedizin Berlin

<120> Determination of renin/prorenin receptor activity

<130> FB17657

<140> EP 06016940.6

<141> 2006-08-14

<160> 59

<170> PatentIn version 3.3

<210> 1
<211> 52
<212> DNA
<213> Homo sapiens

<400> 1
tggatatgat agcatcattt ataggatgac aaaccagaag attcgaatgg at 52

<210> 2
<211> 21
<212> DNA
<213> Homo sapiens

<400> 2
cttaactaca gttttcactg g 21

<210> 3
<211> 26
<212> DNA
<213> Homo sapiens

<400> 3
gccaccatgg ctgtgtttgt cgtgct 26

<210> 4
<211> 47
<212> DNA
<213> Homo sapiens

<400> 4
tcacttgtcg tcatcgtctt tgtagtcatc cattcgaatc ttctggt 47

<210> 5
<211> 53
<212> DNA
<213> Homo sapiens

<400> 5
tcacagatct tcttcagaaa taagtttttg ttcatccatt cgaatcttct ggt 53

<210> 6
<211> 50
<212> DNA
<213> Homo sapiens

<400> 6
tcaagcgtag tctgggacgt cgtatgggta atccattcga atcttctggt 50

<210> 7
<211> 26
<212> DNA
<213> Homo sapiens

<400> 7
gccaccatgg atctgacaaa aatggg 26

<210> 8
<211> 53
<212> DNA
<213> Homo sapiens

<400> 8
tcacagatct tcttcagaaa taagtttttg ttccacatag cacaggtaga ggt 53

<210> 9
<211> 20
<212> DNA
<213> Homo sapiens

<400> 9
ggcaccatgg ctgtgtttgt 20

<210> 10
<211> 19
<212> DNA
<213> Homo sapiens

<400> 10
attcgaatct tctggtttg 19

<210> 11
<211> 25
<212> DNA
<213> Homo sapiens

<400> 11
atggaggcaa aacaagcgaa gaacc 25

<210> 12
<211> 19
<212> DNA
<213> Homo sapiens

<400> 12
attcgaatct tctggtttg 19

<210> 13
<211> 20
<212> DNA
<213> Homo sapiens

<400> 13
ggcaccatgg ctgtgtttgt 20

<210> 14
<211> 23
<212> DNA
<213> Homo sapiens

<400> 14
atccattcga atcctctggt ttg 23

<210> 15
<211> 25
<212> DNA
<213> Homo sapiens

<400> 15
ggtgccgcgg cggccgcagc actgc 25

<210> 16
<211> 25
<212> DNA
<213> Homo sapiens

<400> 16
cttaactaca gttttcactg gaaca 25

<210> 17
<211> 23
<212> DNA
<213> Homo sapiens

<400> 17
ctttacatct gttttcactg gaa 23

<210> 18
<211> 25
<212> DNA
<213> Homo sapiens

<400> 18
tcacagctgg cgtccgtagc cgggc 25

<210> 19
<211> 25
<212> DNA
<213> Homo sapiens

<400> 19
gtgattggtg gagaaagcgg cagct 25

<210> 20
<211> 20
<212> DNA
<213> Homo sapiens

<400> 20
attggcctat accaggagag 20

<210> 21
<211> 20
<212> DNA
<213> Homo sapiens

<400> 21
ttccccataa cgcttcccaa 20

<210> 22
<211> 23
<212> DNA
<213> Homo sapiens

<400> 22
catatttgtc atgattccta ctt 23

<210> 23
<211> 22
<212> DNA
<213> Homo sapiens

<400> 23
gcacaaactg taatattggt gt 22

<210> 24
<211> 20
<212> DNA
<213> Homo sapiens

<400> 24
acatcttcaa cctcgctgtg 20

<210> 25
<211> 20
<212> DNA
<213> Homo sapiens

<400> 25
ccatacacca aacaagggga 20

<210> 26
<211> 20
<212> DNA
<213> Homo sapiens

<400> 26
ctgtggatga aaaggcccta 20

<210> 27
<211> 20
<212> DNA
<213> Homo sapiens

<400> 27
attgcctgta gcctgtcagc 20

<210> 28
<211> 20
<212> DNA
<213> Homo sapiens

<400> 28
gctgcagccc ggcaactttt 20

<210> 29
<211> 20
<212> DNA
<213> Homo sapiens

<400> 29
cggtggagta gatcctgctc 20

<210> 30
<211> 20
<212> DNA
<213> Homo sapiens

<400> 30
tgctcgagat gtgatgaagg 20

<210> 31
<211> 20
<212> DNA
<213> Homo sapiens

<400> 31
tcccctgttg actggtcatt 20

<210> 32
<211> 26
<212> DNA
<213> Homo sapiens

<400> 32
tgaaggtcgg agtcaacgga tttggt 26

<210> 33
<211> 24
<212> DNA
<213> Homo sapiens

<400> 33
catgtgggcc atgaggtcca ccac 24

<210> 34
<211> 20
<212> DNA
<213> Homo sapiens

<400> 34
tccctggaga agagctacga 20

<210> 35
<211> 20
<212> DNA
<213> Homo sapiens

<400> 35
agcactgtgt tggcgtacag 20

<210> 36
<211> 20
<212> DNA
<213> Homo sapiens

<400> 36
ctctcctggt ataggccaat 20

<210> 37
<211> 20
<212> DNA
<213> Homo sapiens

<400> 37
tcggaaaaca acagaccctg 20

<210> 38
<211> 19
<212> RNA
<213> Homo sapiens

<400> 38 19
gcuccguaau cgccuguuu

<210> 39
<211> 19
<212> RNA
<213> Homo sapiens

<400> 39
aaacaggcga uuacggagc 19

<210> 40
<211> 24
<212> DNA
<213> Homo sapiens

<400> 40
catggctgtg tttgtcgtgc tcct 24

<210> 41

<211> 22
<212> DNA
<213> Homo sapiens

<400> 41
tcaatccatt cgaatcttct gg 22

<210> 42
<211> 20
<212> DNA
<213> Homo sapiens

<400> 42
aaacaagcga agaacccagc 20

<210> 43
<211> 21
<212> DNA
<213> Homo sapiens

<400> 43
ggtgataatc acagccaagg c 21

<210> 44
<211> 20
<212> DNA
<213> Homo sapiens

<400> 44
cggatcttgc agagcagttt 20

<210> 45
<211> 20
<212> DNA
<213> Homo sapiens

<400> 45
aggttgctgg agctctgtgt 20

<210> 46
<211> 20
<212> DNA
<213> Homo sapiens

<400> 46
tagggtgcac acaggtgaga 20

<210> 47
<211> 20
<212> DNA
<213> Homo sapiens

<400> 47
gtgcagatgg tgcactggta 20

<210> 48

<211> 23
<212> DNA
<213> Homo sapiens

<400> 48
ccgcagctag gaataatgga ata 23

<210> 49
<211> 20
<212> DNA
<213> Homo sapiens

<400> 49
tctagcggcg caatacgaat 20

<210> 50
<211> 21
<212> DNA
<213> Homo sapiens

<400> 50
cttaactaca gttttcactg g 21

<210> 51
<211> 21
<212> DNA
<213> Homo sapiens

<400> 51
ctttacatct gttttcactg g 21

<210> 52
<211> 21
<212> DNA
<213> Homo sapiens

<400> 52
ttacatgtac tagtgttgtg g 21

<210> 53
<211> 20
<212> DNA
<213> Homo sapiens

<400> 53
gctctgtgcc tcctctctca 20

<210> 54
<211> 19
<212> DNA
<213> Homo sapiens

<400> 54
cccagctgat gaccttgaa 19

<210> 55

<211> 20
<212> DNA
<213> Homo sapiens

<400> 55
aatgctgcac tgtgcggcga 20

<210> 56
<211> 19
<212> DNA
<213> Homo sapiens

<400> 56
ggcggatcgg caaaggcga 19

<210> 57
<211> 20
<212> DNA
<213> Homo sapiens

<400> 57
aaatgaagtg agacccctcc 20

<210> 58
<211> 20
<212> DNA
<213> Homo sapiens

<400> 58
tcaaaggaca cacagcacct 20

<210> 59
<211> 15
<212> DNA
<213> Homo sapiens

<400> 59
aactacagtt ttcac 15

SEQUENCE LISTING

<110> Charité - Universitätsmedizin Berlin

<120> Determination of renin/prorenin receptor activity

<130> FB17657

<160> 59

<170> PatentIn version 3.3

<210> 1
<211> 52
<212> DNA
<213> human

<400> 1
tggatatgat agcatcattt ataggatgac aaaccagaag attcgaatgg at 52

<210> 2
<211> 21
<212> DNA
<213> human

<400> 2
cttaactaca gttttcactg g 21

<210> 3
<211> 26
<212> DNA
<213> human

<400> 3
gccaccatgg ctgtgtttgt cgtgct 26

<210> 4
<211> 47
<212> DNA
<213> human

<400> 4
tcacttgtcg tcatcgtctt tgtagtcatc cattcgaatc ttctggt 47

<210> 5
<211> 53
<212> DNA
<213> human

<400> 5
tcacagatct tcttcagaaa taagtttttg ttcatccatt cgaatcttct ggt 53

<210> 6
<211> 50
<212> DNA
<213> human

<400> 6
tcaagcgtag tctgggacgt cgtatgggta atccattcga atcttctggt 50

<210> 7
<211> 26
<212> DNA
<213> human

<400> 7
gccaccatgg atctgacaaa aatggg 26

<210> 8
<211> 53
<212> DNA
<213> human

<400> 8
tcacagatct tcttcagaaa taagtttttg ttccacatag cacaggtaga ggt 53

<210> 9
<211> 20
<212> DNA
<213> human

<400> 9
ggcaccatgg ctgtgtttgt 20

<210> 10
<211> 19
<212> DNA
<213> human

<400> 10
attcgaatct tctggtttg 19

<210> 11
<211> 25
<212> DNA
<213> human

<400> 11
atggaggcaa aacaagcgaa gaacc 25

<210> 12
<211> 19
<212> DNA
<213> human

<400> 12
attcgaatct tctggtttg 19

<210> 13
<211> 20
<212> DNA
<213> human

<400> 13
ggcaccatgg ctgtgtttgt 20

<210> 14
<211> 23
<212> DNA
<213> human

<400> 14
atccattcga atcctctggt ttg 23

<210> 15
<211> 25
<212> DNA
<213> human

<400> 15
ggtgccgcgg cggccgcagc actgc 25

<210> 16
<211> 25
<212> DNA
<213> human

<400> 16
cttaactaca gttttcactg gaaca 25

<210> 17
<211> 23
<212> DNA
<213> human

<400> 17
ctttacatct gttttcactg gaa 23

<210> 18
<211> 25
<212> DNA
<213> human

<400> 18
tcacagctgg cgtccgtagc cgggc 25

<210> 19
<211> 25
<212> DNA
<213> human

<400> 19
gtgattggtg gagaaagcgg cagct 25

<210> 20
<211> 20
<212> DNA
<213> human

<400> 20
attggcctat accaggagag 20

<210> 21
<211> 20
<212> DNA
<213> human

<400> 21
ttccccataa cgcttcccaa 20

<210> 22
<211> 23
<212> DNA
<213> human

<400> 22
catatttgtc atgattccta ctt 23

<210> 23
<211> 22
<212> DNA
<213> human

<400> 23
gcacaaactg taatattggt gt 22

<210> 24
<211> 20
<212> DNA
<213> human

<400> 24
acatcttcaa cctcgctgtg 20

<210> 25
<211> 20
<212> DNA
<213> human

<400> 25
ccatacacca aacaagggga 20

<210> 26
<211> 20
<212> DNA
<213> human

<400> 26
ctgtggatga aaaggcccta 20

<210> 27
<211> 20
<212> DNA
<213> human

<400> 27
attgcctgta gcctgtcagc 20

<210> 28
<211> 20
<212> DNA
<213> human

<400> 28
gctgcagccc ggcaactttt 20

<210> 29
<211> 20
<212> DNA
<213> human

<400> 29
cggtggagta gatcctgctc 20

<210> 30
<211> 20
<212> DNA
<213> human

<400> 30
tgctcgagat gtgatgaagg 20

<210> 31
<211> 20
<212> DNA
<213> human

<400> 31
tcccctgttg actggtcatt 20

<210> 32
<211> 26
<212> DNA
<213> human

<400> 32
tgaaggtcgg agtcaacgga tttggt 26

<210> 33
<211> 24
<212> DNA
<213> human

<400> 33
catgtgggcc atgaggtcca ccac 24

<210> 34
<211> 20
<212> DNA
<213> human

<400> 34
tccctggaga agagctacga 20

<210> 35
<211> 20
<212> DNA
<213> human

<400> 35
agcactgtgt tggcgtacag 20

<210> 36
<211> 20
<212> DNA
<213> human

<400> 36
ctctcctggt ataggccaat 20

<210> 37
<211> 20
<212> DNA
<213> human

<400> 37
tcggaaaaca acagaccctg 20

<210> 38
<211> 12
<212> RNA
<213> human

<400> 38
gcuccguaau cgccuguuu
12

<210> 39
<211> 17
<212> RNA
<213> human

<400> 39
aaacaggcga uuacggagc 17

<210> 40
<211> 24
<212> DNA
<213> human

<400> 40
catggctgtg tttgtcgtgc tcct 24

<210> 41
<211> 22
<212> DNA

<213> human

<400> 41
tcaatccatt cgaatcttct gg 22

<210> 42
<211> 20
<212> DNA
<213> human

<400> 42
aaacaagcga agaacccagc 20

<210> 43
<211> 21
<212> DNA
<213> human

<400> 43
ggtgataatc acagccaagg c 21

<210> 44
<211> 20
<212> DNA
<213> human

<400> 44
cggatcttgc agagcagttt 20

<210> 45
<211> 20
<212> DNA
<213> human

<400> 45
aggttgctgg agctctgtgt 20

<210> 46
<211> 20
<212> DNA
<213> human

<400> 46
tagggtgcac acaggtgaga 20

<210> 47
<211> 20
<212> DNA
<213> human

<400> 47
gtgcagatgg tgcactggta 20

<210> 48
<211> 23
<212> DNA

<213> human

<400> 48
ccgcagctag gaataatgga ata 23

<210> 49
<211> 20
<212> DNA
<213> human

<400> 49
tctagcggcg caatacgaat 20

<210> 50
<211> 21
<212> DNA
<213> human

<400> 50
cttaactaca gttttcactg g 21

<210> 51
<211> 21
<212> DNA
<213> human

<400> 51
ctttacatct gttttcactg g 21

<210> 52
<211> 21
<212> DNA
<213> human

<400> 52
ttacatgtac tagtgttgtg g 21

<210> 53
<211> 20
<212> DNA
<213> human

<400> 53
gctctgtgcc tcctctctca 20

<210> 54
<211> 19
<212> DNA
<213> human

<400> 54
cccagctgat gaccttgaa 19

<210> 55
<211> 20
<212> DNA

```
<213>  human

<400>  55
aatgctgcac tgtgcggcga                                                   20


<210>  56
<211>  19
<212>  DNA
<213>  human

<400>  56
ggcggatcgg caaaggcga                                                    19


<210>  57
<211>  20
<212>  DNA
<213>  human

<400>  57
aaatgaagtg agacccctcc                                                   20


<210>  58
<211>  20
<212>  DNA
<213>  human

<400>  58
tcaaaggaca cacagcacct                                                   20

<210>  59
<211>  20
<212>  DNA
<213>  human

<400>  59
aactacagtt ttcac                                                        15
```

## Description

The present invention refers to the renin/prorenin receptor (RER) signal transduction pathway and, in particular, to the role of promyelocytic zinc finger protein (PLZF) and its downstream targets involved in this pathway. In more detail, the present invention refers to a method for determining RER activity, e.g. stimulation or inhibition of RER activity, using PLZF activity as a measurement from which RER activity is derived. The present invention further refers to a use of said method for identifying RER ligands, e.g. pharmaceutically active agonists or antagonists, as well as for studying undesired side-effects of renin inhibitors.

## Background of the Invention

Hypertension and diabetes are chronic diseases with particular high incidence in Western countries. Although effective therapies are available today, the development and progression of severe end organ damages such as diabetic nephropathy, retinopathy and heart failure associated with these diseases remain serious problems.

Amongst the hormones which are involved in the pathophysiology of hypertension and diabetes, renin and its precursor prorenin play important roles. Renin and prorenin are classically thought of as (pro)enzymes of the renin-angiotensin system (RAS), but recent evidence suggests that they also act as hormones because of their ability to bind cellular targets (Re, 2003). In 2002 a human renin/prorenin receptor (RER) has been cloned, which consists of 350 amino acids with a single transmembrane domain and specifically binds prorenin and renin. Interestingly, this receptor exerts a dual molecular function (Nguyen *et al.,* 2004; Nguyen *et al.,* 2002): (1) Binding of renin to its receptor increases renin's catalytic activity about 4- to 5-fold. Furthermore, prorenin, which does not exhibit significant ability to generate angiotensin I in solution, gains enzymatic activity comparable to renin by binding to the RER, i.e., the receptor is able to unmask the catalytic activity of prorenin. (2) The RER is also able to induce a signal transduction cascade upon ligand binding. Binding of renin and also prorenin causes phosphorylation of the receptor and activation of the MAP (mitogen-activated protein) kinases ERK1 and ERK2, whereas intracellular calcium or cAMP levels are not altered. Remarkably, even deglycosylated renin is able to induce ERK1/2 phosphorylation (Mazak *et al.,* 2003).

The mRNA of RER is highly expressed in brain, heart and placenta with highest levels in the brain. In contrast, kidney, liver and pancreas show low mRNA expression levels (Nguyen *et al.,* 2004; Nguyen *et al.,* 2002). Via immunofluorescence, the receptor has been detected in mesangial and vascular smooth muscle cells of human heart and kidney (Nguyen *et al.,* 2004; Nguyen *et al.,* 2002). In addition, mRNA and protein expression have been demonstrated in macrophages, T-cells and granulocytes (Mazak *et al.,* 2003). The receptor is expressed on the cell surface in transfected mesangial cells (Nguyen *et al.,* 2002), but there are also indications of an (additional) intracellular receptor localization (Mazak *et al.,* 2003; Nguyen *et al.,* 2004).

The cloned RER probably corresponds to the previously identified renin binding site on mesangial cells implicated in regulation of the plasminogen activator inhibitor-1 (PAI-1) and hypertrophic effects (Nguyen *et al.,* 2002; Nguyen *et al.,* 1996). Very recently, it was shown that transgenic overexpression of the RER in smooth muscle cells causes a blood pressure elevation and an increase in heart rate (Burckle *et al.,* 2006).

Concerning proteins encoded by the RER gene, it is of interest, that the C-terminal part of the RER is identical to the vacuolar proton-translocating ATPase (V-ATPase) membrane sector-associated protein M8-9 (APT6M8-9 = ATP6AP2) (Ludwig *et al.,* 1998; Ramser *et al.,* 2005). V-ATPases, in general, exert several cellular functions such as neurotransmitter uptake and storage, endocytosis and receptor recycling (Nelson and Harvey, 1999). Furthermore, RER and CAPER (homo sapiens ER-localized type I transmembrane adaptor precursor) are identical transcripts (GenBank accession number AY038990).

With regard to the biological significance of the RER beyond its possible role in the RAS, it was recently shown by Ramser and colleagues that a mutation in the renin receptor gene is a cause of X-linked mental retardation (XLMR) and epilepsy (XMRE) syndrome in humans (Ramser *et al.,* 2005). Consistent with this observation are the results of a zebrafish mutagenesis screen, in which a mutation in the ATP6AP2 (= RER) gene caused a reduction in head size and necrosis of the central nervous system (CNS) (Amsterdam *et al.,* 2004). Remarkably, the human RER mutation observed by Ramser and colleagues neither altered binding affinity for renin nor the RER-mediated augmentation of renin's catalytic efficiency of angiotensinogen cleavage (Ramser *et al.,* 2005). In addition, the effect of this mutation - and also the effect of wild type RER stimulation by renin (Nguyen *et al.,* 2002) - on MAPK signalling are only modest (Ramser *et al.,* 2005), if not insignificant.

Related to the role of the RER in CNS development is the observation, that RER mRNA can be detected in human glioblastomas as well as in glioblastoma cell lines, and that renin inhibitors can reduce the cell number in glioblastoma cell lines. This is probably caused by modulation of RER function, since this effect is independent of angiotensin AT1 and AT2 receptor activity (Juillerat-Jeanneret *et al.,* 2004).

The clinical relevance of the RER is underlined by the interesting observation that a decoy deca-peptide corresponding to the handle region of prorenin, which competitively inhibits prorenin binding to its receptor, attenuated the development and progression of cardiac fibrosis (Ichihara *et al.,* 2005), and also inhibited the development of diabetic nephropathy in rat models (Ichihara *et al.,* 2004).

Considering the pathophysiologic relevance of the RER, it would be of particular interest to provide insights into the RER signal transduction pathway. Such knowledge would be useful, for example, in the development of pharmaceutically active RER agonists and antagonists.

Moreover, knowledge regarding the signal transduction of the RER would be of importance to evaluate the efficacy and safety of renin inhibitors, such as aliskiren, which is currently in phase III clinical trials (Hershey *et al.,* 2005). As expected, renin inhibitors reduce plasma renin activity (i.e., enzyme activity with respect to angiotensin I generation). Nevertheless, they increase total amount of plasma renin protein - the RER ligand - dramatically (up to 34-fold; Nussberger *et al.,* 2002). Therefore, it is crucial to examine whether renin inhibitors change the intrinsic activity of renin with respect to the RER. In this context, it is important to note that over-activation of the RER might be deleterious (e.g., with respect to end-organ damage) considering the activation of MAP kinases and other previously unknown interaction partners downstream of the RER as well as the effects of the handle region decoy peptide mentioned above. On the other hand, a blockade of the RER signal transduction might be harmful, at least in pregnant women, because of the developmental importance of the RER.

Thus, in view of the above, it is an object of the present invention to provide a method and molecular tools allowing for studies on the physiological and pathophysiological occurrences associated with RER activity, for the development of pharmaceutically relevant RER agonists and antagonists, and for the evaluation of renin inhibitor side-effects.

**Summary of the Invention**

The object of the present invention is solved by a method for determination of renin/prorenin receptor (RER) activity, wherein promyelocytic zinc finger protein (PLZF) activity is used as a measurement for RER activity.

In one embodiment, a stimulation of RER activity is detected by RER/PLZF protein interaction and/or PLZF translocation and/or PLZF recruitment.

The object of the present invention is further solved by a method for determination of RER activation, wherein PLZF activation is used a measurement for RER activation.

In one embodiment, RER activation is detected by RER/PLZF protein interaction and/or PLZF translocation and/or PLZF recruitment.

The object of the present invention is further solved by a method for determination of RER inhibition, wherein PLZF inhibition is used as a measurement for RER inhibition.

In one embodiment, RER inhibition is detected by an inhibition of RER/PLZF protein interaction and/or PLZF translocation and/or PLZF recruitment.

In one embodiment, RER/PLZF protein interaction is determined by a method comprising co-immunoprecipitation (coIP), and optionally Western blot analysis.

In one embodiment, RER/PLZF protein interaction involves an interaction domain comprising the cytoplasmatic C-terminal tail of RER.

In one embodiment, the interaction domain comprises an amino acid sequence encoded by a DNA sequence according to SEQ ID No. 1.

The DNA sequence according to SEQ ID No. 1 reads as follows:

TGG ATA TGA TAG CAT CAT TTA TAG GAT GAC AAA CCA GAA GAT TCG AAT GGA T

which DNA sequence corresponds to the last eighteen C-terminal amino acids of RER.

In one embodiment, PLZF translocation is detected by a decrease in cytoplasmatic PLZF protein and/or an increase in nuclear PLZF protein.

In one embodiment, the detected PLZF protein is natural protein in a native or denatured condition, and the protein is detected by an antibody.

In one embodiment, the detected PLZF protein comprises a tag selected from c-myc, FLAG, and HA, and the tag is detected by an antibody, which antibody may carry a fluorescent label.

In one embodiment, the detected PLZF protein comprises a fluorescent tag, preferably enhanced green fluorescent protein (EGFP).

In one embodiment, the decrease in cytoplasmatic PLZF protein and/or the increase in nuclear PLZF protein is determined by a method comprising fractionated extraction of cytosolic and/or nuclear proteins, and optionally Western blot analysis.

In one embodiment, the decrease in cytoplasmatic PLZF protein and/or the increase in nuclear PLZF protein is determined by a method comprising a cytology or histology based procedure.

In one embodiment, the method further comprises fluorescence or immunofluorescence microscopy.

In one embodiment, the decrease in cytoplasmatic PLZF protein and/or the increase in nuclear PLZF protein is determined by a method comprising an immunocytology or immunohistology based procedure and immunofluorescence microscopy.

In one embodiment, PLZF recruitment is detected by binding of PLZF protein to a RER promoter region.

In one embodiment, binding of PLZF protein to the RER promoter region involves a PLZF cis-element.

In one embodiment, binding of PLZF protein to the RER promoter region involves a DNA sequence comprising a DNA sequence according to SEQ ID No. 2.

The DNA sequence according to SEQ ID No. 2 reads as follows:

5'- CTT AAC TAC AGT TTT CAC TGG -3'

In one embodiment, binding of PLZF protein to the RER promoter region is determined by a method comprising chromatin-immunoprecipitation (ChIP), and optionally real-time PCR analysis.

In one embodiment, binding of PLZF protein to the RER promoter region is determined by a method comprising an electromobility shift assay (EMSA).

In one embodiment, PLZF recruitment is detected by a decrease in RER mRNA.

In one embodiment, the decrease in RER mRNA is determined by a method comprising real-time PCR, Northern blot analysis or a microarray technique.

In one embodiment, PLZF recruitment is detected by a decrease in RER protein.

In one embodiment, the decrease in RER protein is determined by a method comprising an immunology based procedure such as Western blot analysis, enzyme-linked immunoabsorbent assay (ELISA), or radioimmuno assay (RIA).

In one embodiment, PLZF recruitment is detected by a decrease in RER promoter activity.

In one embodiment, the decrease in RER promoter activity is determined by a method comprising a reporter gene assay, preferably a luciferase reporter gene assay.

In one embodiment, PLZF recruitment is detected by an increase in the p85$\alpha$ subunit of phosphatidylinositol-3 kinase (PI3K-p85$\alpha$) mRNA.

In one embodiment, the increase in PI3K-p85$\alpha$ mRNA is determined by a method comprising real-time PCR analysis, Northern blot analysis or a microarray technique.

In one embodiment, PLZF recruitment is detected by a decrease in PI3K-p85$\alpha$ protein.

In one embodiment, the decrease in PI3K-p85$\alpha$ protein is determined by a method comprising an immunology based procedure such as Western blot analysis, ELISA, or RIA.

In one embodiment, PLZF recruitment is detected by an increase in PI3K-p85$\alpha$ promoter activity.

In one embodiment, the increase in PI3K-p85$\alpha$ promoter activity is determined by a method comprising a reporter gene assay, preferably a luciferase reporter gene assay.

The object of the present invention is further solved by a RER/PLZF protein interaction domain comprising an amino acid sequence encoded by a DNA sequence according to SEQ ID No. 1.

The object of the present invention is further solved by a PLZF/RER promoter interaction region comprising a DNA sequence according to SEQ ID No. 2.

The object of the present invention is further solved by a use of the method according to the present invention for identifying a RER ligand.

In one embodiment, the RER ligand is a RER antagonist or agonist.

In one embodiment, the RER ligand is a pharmaceutically active agent.

In one embodiment, the RER ligand is selected from a protein, a peptide, a small molecule, a decoy or a peptide decoy.

In one embodiment, the RER ligand is a complex comprising a renin and/or prorenin and a renin inhibitor.

The object of the present invention is further solved by a use of the method according to the present invention for screening a compound library comprising at least one putative RER ligand.

In one embodiment, the putative RER ligand is a RER antagonist or agonist.

In one embodiment, the putative RER ligand is a pharmaceutically active agent.

In one embodiment, the putative RER ligand is selected from a protein, a peptide, a small molecule, a decoy or a peptide decoy.

The object of the present invention is further solved by a use of the method according to the present invention for studying the effect of a RER ligand.

In one embodiment, the RER ligand is a RER antagonist or agonist.

In one embodiment, the RER ligand is a pharmaceutically active agent.

In one embodiment, the RER ligand is selected from a protein, a peptide, a small molecule, a decoy or a peptide decoy.

In one embodiment, the RER ligand is a complex comprising renin or prorenin and a renin inhibitor.

In one embodiment, the studied effect of the RER ligand is an undesired side-effect.

The object of the present invention is further solved by a use of the method according to the present invention for studying undesired renin inhibitor side-effects.

The object of the present invention is further solved by a use of an amino acid sequence encoded by a DNA sequence according to SEQ ID No. 1 or a part or a derivative thereof for determining RER activity.

The object of the present invention is further solved by a use of an amino acid sequence encoded by a DNA sequence according to SEQ ID No. 1 or a part or a derivative thereof for studying RER signal transduction.

The object of the present invention is further solved by a use of a DNA sequence according to SEQ ID No. 1 or a part or a derivative thereof for determining RER activity.

The object of the present invention is further solved by a use of a DNA sequence according to SEQ ID No. 1 or a part or a derivative thereof for studying RER signal transduction.

The object of the present invention is further solved by a use of a DNA sequence according to SEQ ID No. 2 or a part or a derivative thereof for determining RER activity.

The object of the present invention is further solved by a use of a DNA sequence according to SEQ ID No. 2 or a part or a derivative thereof for studying RER signal transduction.

The object of the present invention is further solved by a RNA sequence encoded by a DNA sequence according to SEQ ID No. 2 or a part or a derivative thereof for determining RER activity.

The object of the present invention is further solved by a RNA sequence encoded by a DNA sequence according to SEQ ID No. 2 or a part or a derivative thereof for studying RER signal transduction.

The wording "determination of RER activity" means that both a stimulation of RER activity, i.e. RER activation, and an inhibition of RER activity can be subject of the determination. A stimulation of RER activity may be initiated upon binding or occupation of RER by an agonist. An inhibition of RER activity comprises an inhibition or prevention or blockade of RER activation.

The terms "determination" and "determined" refers both to a qualitative and quantitative determination.

The wording "PLZF activity is used as a measurement for RER activity" means that in the determination of RER activity, PLZF activity, e.g. stimulation or inhibition of PLZF activity, is actually measured, and the information obtained for PLZF activity serves as a basis on which RER activity is assessed. In other words, PLZF activity is used as an indicator of RER activity. In this regard, PLZF and RER are in a relationship such that a stimulation of PLZF activity points to a stimulation of RER activity and, vice versa, an inhibition of PLZF activity points to an inhibition of RER activity.

The wording "cytology based procedure" refers to a procedure involving cells, preferably whole cells. The wording "histology base procedure" refers to a procedure involving tissues. The wording "immunology based procedure" refers to a procedure involving antibodies.

The term "RER ligands" refers to any binding compounds such as endogenous compounds, e.g. renin and prorenin, or exogenous compounds, e.g. pharmaceutically active agents. In one embodiment, "RER ligands" refer to RER agonists, i.e. RER activators, e.g. renin and prorenin. Putative indications for pharmaceutically active RER agonists comprise epilepsy, mental retardation and dementia. In an alternative embodiment, "RER ligands" refer to RER antagonists, also referred to as RER blockers, and sometimes also referred to as RER inhibitors. Putative indications for RER antagonists comprise hypertension, endorgan damage and diseases related to proliferation and fibrosis. Also comprised by "RER ligands" are complexes comprising renin or prorenin and inhibitors of their enzymatic activities.

In summary, the present study describes a novel signal transduction cascade involving interaction of the RER with the transcription factor PLZF. No direct protein interaction partner of the RER has been described so far.

A human RER, which is involved in brain development and cardiac and renal end-organ damage, has recently been cloned (see above). To gain insight into the molecular function of the RER, we studied its signal transduction mechanisms. We could identify the transcription factor PLZF as direct protein interaction partner of the RER by yeast two-hybrid screening and co-immunoprecipiation. A RER interaction domain was identified by IP mapping (see also SEQ ID No. 1). Upon activation of the RER by renin, PLZF is translocated into the nucleus, represses transcription of the RER itself, thereby creating a very short negative feedback loop, but activates transcription of PI3K-p85$\alpha$. siRNA against the RER abolished these effects. A PLZF cis-element in the RER promoter was identified by site-directed mutagenesis and EMSA (see also SEQ ID No. 2). Additionally, renin stimulation caused a 6-fold recruitment of PLZF to this promoter region as shown by chromatin-immunoprecipitation.

To conclude, our results demonstrate the existence of a novel signal transduction pathway downstream of the human RER, which involves direct binding of the transcription factor PLZF to the receptor, its translocation to the nucleus and the positive and negative regulation of target genes. Based on the already described biomedical relevance of the RER and PLZF, respectively, this pathway - connecting both molecules - might be of importance in human physiology and pathophysiology.

Moreover, our results provide PLZF as a target of measurement for the determination of RER activity whereupon signal transduction downstream from the receptor is initiated. To briefly summarize, for determination of RER activity, use can be made of the following stages of the RER-PLZF signal transduction pathway: (1) RER protein/PLZF protein interaction, (2) PLZF protein translocation, and (3) PLZF protein/DNA interaction. The latter can be separated into two sub-stages, namely (3a) interaction of PLZF protein with the RER promoter leading to a decrease in promoter activity and, as a result, decreased mRNA and protein, and (3b) interaction of PLZF protein with the PI3K-p85α promoter leading to an increase in promoter activity and, as a result, increased mRNA and protein.

The method of the present invention allows for the identification of RER ligands without knowledge of the receptor's molecular structure. Compared to the ERK1/2 signal transduction pathway activated by RER, the RER signal transduction pathway involving PLZF turned out to be less susceptible to erroneous signals.

Thus, in addition to studies on RER signal transduction mechanisms, the method of the present invention allows for the development of RER agonists and antagonists for pharmaceutical purposes and for the evaluation of undesirable side-effects of renin inhibitors.

Detailed Description of the Invention

Figure 1A shows the results of a RT-PCR expression analysis of the human RER and other components of the RAS. The indicated cell lines and tissues of human origin were analysed by RT-PCR. HPRT, GAPDH and β-actin served as positive controls. A reaction without addition of reverse transcriptase (RT) and a no template control (NTC) served as negative controls (data not shown). AT1R: angiotensin II type 1 receptor; AT2R: angiotensin II type 2 receptor; AGT: angiotensinogen; ACE: angiotensin-converting enzyme.

Figure 1B shows the results of a Northern blot analysis of the human RER.
Total RNA extracted from the indicated human cells and from human kidney were hybridized with probes specific for human RER. Standardization was performed against human β-actin.

Figure 2A shows the transcriptional start sites of the human RER.
A RNA ligase-mediated-5'-RACE was performed on SH-SY5Y cells, and the resulting PCR products were separated in an agarose gel (left side) and subcloned for sequencing (right side). 1: 100 bp DNA ladder; 2: RACE reaction; 3: negative control. Transcriptional start sites are boxed and underlined; the translational start site "atg" is marked bold. The sequence shown corresponds to GenBank GI:37546587.

Figure 2B shows a characterization of the human RER promoter in different human cells. Serial deletion mutants of the human RER promotor were subcloned into the pGL3-basic luciferase reporter vector. Numbers indicate bp upstream from the translational start site. Standardization was achieved by cotransfection with phRL-null vector, which encodes *Renilla* luciferase. Relative luciferase activity (RLA) indicates promoter activity relative to an insertless pGL3-basic vector. The strong housekeeping promoter of the human ECE-1c isoform (968 bp upstream of the translational start site) served as control (C).

Figure 3A shows the result of a Western blot analysis of the human RER receptor. Cytosolic (C) (12 μg) and membrane (M) (9 μg) proteins of HeLa-S3 cells were separated by SDS-PAGE and Western blotting was performed using an antibody against human RER.

Figure 3B shows the result of immunocytologic analyses of full-length RER.
HeLa-S3 cells were transiently transfected with c-myc- and FLAG-tagged full-length RER, and subcellular localization was analysed by immunofluorescence microscopy. Transfection of an insertless vector served as negative control. DAPI was used as nuclear marker.

Figure 3C shows the results of fluorescence microscopy studies of EGFP-RER fusion proteins.
A full-length wild type RER (RER full), a full-length RER, in which the atypical endoplasmic reticulum (ER)-retention motif has been mutated (RER K/R mut), and the V-ATPase segment of the RER (RER ATPase), each C-terminally fused to EGFP, were transiently transfected into HeLa-S3 cells. Markers for the nuclear (DAPI), ER and lysosomal compartment were used as indicated.

Figure 4A shows the results of a RER-PLZF interaction analysis by coimmunoprecipitation (coIP).
HEK293 cells were transiently transfected with FLAG-tagged RER and/or c-myctagged PLZF. Immunoprecipitation and Western blotting were performed using anti-FLAG and anti-c-myc antibodies. Several controls were employed as indicated.

Figure 4B shows the results of an analysis of endogenous interaction between RER and PLZF.
Total lysate of HEK293 cells was subjected to immunoprecipitation using an anti-PLZF antibody or protein A agarose as control. Subsequent Western blotting of total lysate and IP eluates were performed as indicated.

Figure 4C shows the cytoplasmatic C-terminal tail of the RER as interaction domain.
HEK293 cells were cotransfected with pCEP4-PLZF-myc and deletion mutants of the RER fused to EGFP (RER full: full-length CDS; RER n-term del: deletion of the first N-terminal 16aa; RER c-term del: deletion of the cytoplasmic (C-terminal) tail; EGFP alone). Co-immunoprecipitation was performed with an agarose-coupled anti-myc-antibody. Total lysates and IP eluates have been subjected to Western blotting.

Figure 4D shows that the RER is able to form homodimers.
The human RER was tagged with two different tags and transiently transfected into HEK293 cells. The ability of the RER to form homodimers was shown by co-immunoprecipitation (coIP).

Figure 5A shows an decrease of RER mRNA by renin stimulation and PLZF cotransfection.
HEK293 cells with (pCEP4-PLZF; +) or without (pCEP4 insertless; -) PLZF overexpression were stimulated with renin or vehicle, and RER mRNA was quantified by real-time PCR. mRNA expression level of the first column was set to 100%.

Figure 5B shows that PLZF is able to repress RER promoter activity.
SH-SY5Y and HEK293 cells were transfected with serial deletion mutants of human RER promoter (numbers indicate promoter region relative to the translation start site in bp). Cotransfection was performed with a PLZF expression vector (hatched columns) or an insertless control vector (pCEP4; white columns). RER promoter activity was determined using a luciferase reporter assay. RLA: relative luciferase activity.

Figure 6A shows that a repression of RER promoter activity by renin stimulation requires RER and a PLZF cis-element.
HEK293 cells overexpressing PLZF (by transient transfection of a pCEP4-PLZF expression vector) were stimulated with renin (hatched columns) or vehicle (white columns), and relative luciferase activity (RLA) of a wild type RER promoter reporter construct (WT) or a RER promoter reporter construct, with a mutation of the PLZF consensus sequence at position [-1097; -1083] (mut), was determined. -1110 indicates the length of the subcloned promoter relative to the ATG. The functional importance of the RER was examined by using siRNA against RER (siRNA +) compared to a control siRNA (siRNA -).

Figure 6B shows that an activation of PI3K-p85$\alpha$ promoter activity by renin stimulation requires RER.
The activity of the PI3K-p85$\alpha$ promoter was analysed in PLZF overexpressing HEK293 cells in an experimental setting analogous to Figure 6A, using siRNA against the RER or control siRNA.

Figure 6C shows that an increase of PI3K-p85$\alpha$ mRNA by renin stimulation requires RER.
HEK293 cells with (pCEP4-PLZF) or without (pCEP4 insertless) PLZF overexpression were stimulated with renin (hatched columns) or vehicle (white columns), and PI3K-p85a mRNA (normalized to 18S rRNA) was determined by quantitative realtime PCR. The functional importance of the RER was analysed by siRNA against RER (siRNA +) compared to a control siRNA (siRNA -). mRNA expression level of the first column was set to 100%.

Figure 6D shows that RER siRNA reduced RER protein.
Cells were treated with siRNA against the RER or control siRNA, respectively. 10 $\mu$g of total cell lysate have been subjected to Western blotting against native RER and GAPDH.

Figure 7A shows that RER activation causes a translocation of PLZF to the nucleus.
HEK293 were transfected with c-myc-tagged PLZF. 24 h later, siRNA directed against the RER (+), or a siRNA control (-), were applied. 48 h after siRNA transfection, cells were incubated with 10 nM renin for 1 h after a 24 h starving period, followed by fractionated isolation of cytosolic and nuclear proteins. These proteins were subjected to SDS-PAGE and Western blotting. A Western blot directed against TATA box-binding protein (TBP) served as control for subcellular fractionation and loading.

Figure 7B also shows that RER activation causes a translocation of PLZF to the nucleus.
The experiment has been performed according to a procedure underlying Figure 7A above, but without any transfections. Western blot detection was performed against native PLZF.

Figure 7C shows that renin stimulation increased recruitment of PLZF to the RER promoter.
HEK293 cells were stimulated for 2 hours with renin or vehicle followed by ChIP using an anti-PLZF antibody. Recruitment of PLZF to the RER promoter was analysed by real-time PCR and standardized to the recruitment of TBP to the β-actin promoter.

Figure 7D shows that PLZF protein binds to a consensus sequence within the human RER promoter.
Nuclear proteins were extracted from native HEK293 cells. An electromobility shift assay (EMSA) was performed using oligonucleotides corresponding to the wild type and a mutated form of the PLZF consensus sequence at position [-1097; -1083] in the human RER promoter. An oligonucleotide derived from the PI3K-p85α promoter served as control. S: specific band shift; SS1, SS2: super-shifted band shift; ns: nonspecific band shift.

Figure 8 schematically depicts the signal transduction pathway of the RER.

EXAMPLES

EXAMPLE 1: Materials and methods

*Cell culture*

SH-SY5Y (human neuronal) and SK-N-AS (human neuronal) were cultured in RPMI 1640 supplemented with 10% fetal bovine serum (FBS), 100 U/ml penicillin and 100 μg/ml streptomycin. HEK293 (human epithelial), HeLa-S3 (human epithelial), EA.hy926 (human endothelial), T98G (human glial), U-87 MG (human glial), and U-373 MG (human glial) were cultured in DMEM with 4,5 g/l glucose supplemented with 10% FBS, 100 U/ ml penicillin and 100 μg/ ml streptomycin. All cell culture products were obtained from PAN Biotech, Aidenbach, Germany. Cells were grown in a humidified incubator at 5% $CO_2$ and 37 °C.

*Constructs and site-directed mutagenesis*

The complete coding sequence (CDS) of the human RER, based on GenBank GI:21325928, and human PLZF, based on GenBank GI:31543978, were subcloned into the mammalian expression vector pCEP4 (Invitrogen, Karlsruhe, Germany) with different C-terminal tags using the following primers:

5'-GCCACCATGGCTGTGTTTGTCGTGCT (sense for pCEP4-RER-FLAG/-myc/-HA), (SEQ ID No. 3);
5'-TCACTTGTCGTCATCGTCTTTGTAGTCATCCATTCGAATCTTCTGGT (antisense for pCEP4-RER-FLAG), (SEQ ID No. 4);
5'-TCACAGATCTTCTTCAGAAATAAGTTTTTGTTCATCCATTCGAATCTTCTGGT (antisense for pCEP4-RER-myc), (SEQ ID No. 5);
5'-TCAAGCGTAGTCTGGGACGTCGTATGGGTAATCCATTCGAATCTTCTGGT (antisense for pCEP4-RER-HA), (SEQ ID No. 6);
5'-GCCACCATGGATCTGACAAAAATGGG (sense for pCEP4-PLZF-myc), (SEQ ID No. 7);
5'-TCACAGATCTTCTTCAGAAATAAGTTTTTGTTCCACATAGCACAGGTAGAGGT (antisense for pCEP4-PLZF-myc), (SEQ ID No. 8).

Additionally, the CDS of the human RER was cloned into pEGFP-N1 and pEGFP-C3 (Clontech, Mountain View, CA, USA) to create N- or C-terminal EGFP fusion constructs using the following primers:

5'-GGCACCATGGCTGTGTTTGT (sense for RER-full), (SEQ ID No. 9);
5'-ATTCGAATCTTCTGGTTTG (antisense for RER-full), (SEQ ID No. 10).

Furthermore, two protein mutants of the RER were generated:

(1) The pEGFP-N1/C3-RER-ATPase construct, which comprises only the vacuolar proton-translocating ATPase (V-ATPase) membrane sector-associated protein M8-9 (APT6M8-9) part of the RER (C-terminal 69 aa), was subcloned using the following primers:

5'-ATGGAGGCAAAACAAGCGAAGAACC (sense primer), (SEQ ID No. 11);
5'-ATTCGAATCTTCTGGTTTG (antisense primer), (SEQ ID No. 12).

(2) The pEGFP-N1/C3-RER-K/R-mut construct, in which the atypical C-terminal ER-retention signal (KXXXX) was replaced by RXXXX, was subcloned using the following primers:

5'-GGCACCATGGCTGTGTTTGT (sense primer), (SEQ ID No. 13);
5'-ATCCATTCGAATCCTCTGGTTTG (antisense primer), (SEQ ID No. 14).

The promoter of the human RER, based on GenBank GI:37546587, and serial deletion mutants were subcloned into the luciferase reporter vector pGL3-basic (Promega, Mannheim) using a common antisense primer located directly upstream of the translational start site (5'-GGTGCCGCGGCGGCCGCAGCACTGC; SEQ ID No. 15) and following sense primers (numbers indicate positions relative to the start codon):

[-1100;-1]: 5'-CTTAACTACAGTTTTCACTGGAACA (SEQ ID No. 16);
[-1100;-1]-mut(PLZF): 5'-CTTTACATCTGTTTTCACTGGAA (SEQ ID No. 17);
[-500;-1]: 5'-TCACAGCTGGCGTCCGTAGCCGGGC (SEQ ID No. 18);
[-165;-1]: 5'-GTGATTGGTGGAGAAAGCGGCAGCT (SEQ ID No. 19).

A pGL3-basic plasmid encoding 968 bp of the promoter of human endothelin-converting enzyme-1c (ECE-1c) (Funke-Kaiser *et al.,* 2003c) was used as control.

*RT-PCR*

RNA of human heart and tissue of human kidney were provided by the DHZB (Berlin) and the Department of Urology (Charité - Universitatsmedizin Berlin), respectively. Total RNA was isolated using NucleoSpin RNA II (Macherey-Nagel, Düren, Germany) according to the manufacturer's protocol including a DNAase digest. cDNA synthesis was performed using random hexamer primers and M-MLV reverse transcriptase (RNase H minus; Promega, Mannheim, Germany); no template controls (NTCs) and reactions without addition of reverse transcriptase (RT-) served as negative controls.

PCRs were carried out using the following human-specific primer pairs:

RER: 5'-ATTGGCCTATACCAGGAGAG (sense), (SEQ ID No. 20),
5'-TTCCCCATAACGCTTCCCAA (antisense), (SEQ ID No. 21);
angiotensin AT1 receptor (AT1R): 5'-CATATTTGTCATGATTCCTACTT (sense),
(SEQ ID No. 22),
5'-GCACAAACTGTAATATTGGTGT (antisense), (SEQ ID No. 23);
angiotensin AT2 receptor (AT2R): 5'-ACATCTTCAACCTCGCTGTG (sense),
(SEQ ID No. 24),
S'-CCATACACCAAACAAGGGGA (antisense), (SEQ ID No. 25);
angiotensinogen: 5'-CTGTGGATGAAAAGGCCCTA (sense), (SEQ ID No. 26),
5'-ATTGCCTGTAGCCTGTCAGC (antisense), (SEQ ID No. 27);
ACE: 5'-GCTGCAGCCCGGCAACTTTT (sense), (SEQ ID No. 28),
5'-CGGTGGAGTAGATCCTGCTC (antisense), (SEQ ID No. 29);
HPRT: 5'-TGCTCGAGATGTGATGAAGG (sense), (SEQ ID No. 30),
5'-TCCCCTGTTGACTGGTCATT (antisense), (SEQ ID No. 31);
GAPDH: 5'-TGAAGGTCGGAGTCAACGGATTTGGT (sense), (SEQ ID No. 32);
5'-CATGTGGGCCATGAGGTCCACCAC (antisense), (SEQ ID No. 33); β-actin: 5'-TCCCTGGAGAAGAGCTACGA (sense), (SEQ ID No. 34),
5'- AGCACTGTGTTGGCGTACAG (antisense), (SEQ ID No. 35).

*Northern blotting*

Northern blotting was performed as described previously (Orzechowski *et al.,* 2001; Funke-Kaiser *et al.,* 2003a).

The probe against the human RER and human β-actin (for standardization) corresponds to the PCR products described above.

*RNA ligase-mediated (RLM)- 5'-RACE*

Transcriptional start sites of human RER were determined using the GeneRacer Kit (Invitrogen, Karlsruhe, Germany), which only amplifies capped mRNA. Reverse transcription was performed with random hexamer primers. Nested PCR utilized HotStarTaq (Qiagen, Hilden, Germany), 5'-ctctcctggtataggccaat (antisense primer in first PCR; SEQ ID No. 36) and 5'-tcggaaaacaacagaccctg (antisense primer in second PCR; SEQ ID No. 37). Reaction products were subcloned and sequenced.

*Luciferase assays*

The indicated cell types were seeded on day 1 in 12-well plates. 100 ng of indicated pGL3-basic constructs (encoding firefly luciferase) and 20 ng of phRL-null plasmid (encoding humanized *Renilla* luciferase for standardization of promoter activity; Promega) per well were transfected on day 2 at 60-80% confluency using GeneJuice (Merck Biosciences, Bad Soden, Germany) according to the manufacturer's protocol. Cells were harvested 48 hours after transfection using Passive Lysis Buffer (Promega). Reporter activities were measured in a Pharmingen Monolight 3010 luminometer (BD Biosciences, Erembodegem, Belgium) using the Dual-Luciferase Reporter Assay System (Promega). Promoter activity is expressed as relative luciferase activity (RLA) (Funke-Kaiser *et al.,* 2003a). RLA data represent the mean ± standard deviation of at least three single, parallel transfection experiments.

*Subcellular protein extraction and Western blotting*

Cytosolic and membrane proteins were isolated using the ProteoExtract Subcellular Proteome Extraction Kit (Calbiochem, Darmstadt, Germany). A fractionated extraction of cytosolic and nuclear proteins was performed as described previously (Funke-Kaiser *et al.,* 2003a). Protein concentrations were determined using the DC Protein Assay (Bio-Rad, München, Germany). Cytosolic, membrane and nuclear fractions were controlled by Western blot using antibodies against Akt (mouse monoclonal, 5G3; 1:1,000; Cell Signalling, Danvers, MA, USA), angiotensin AT1 receptor (rabbit polyclonal, sc-579; 1:2,000; Santa Cruz Biotechnology, Heidelberg, Germany) and TBP (rabbit polyclonal, sc-204; 1:1,000; Santa Cruz Biotechnology), respectively.

For analysis of subcellular localization of RER, 9 μg (membrane) and 12 μg (cytosolic) protein were loaded per lane, separated in a 10% SDS-PAGE and transferred to a nitrocellulose membrane. Western blot analysis was performed with an antibody against human RER (corresponding ATP6AP2; goat polyclonal, ab5959, Abcam, Cambridge, UK) at a dilution of 0.5 ng/ ml. Horseradish peroxidase-conjugated anti-goat antibody (rabbit polyclonal; 1:2,500; DAKO, Hamburg, Germany) was used as secondary antibody.

For PLZF translocation experiments HEK293 cells were seeded on day 1 in 6-well plates. 250 ng of pCEP4-PLZF-myc expression vector per well were transfected on day 2 at 40-60% confluency using GeneJuice (Merck Biosciences, Bad Soden, Germany) according to the manufacturer's protocol. On day 3 the cells were transfected with 50 nM siRNA against the RER [5'-GCUCCGUAAUCGCCUGUUU (sense strand), (SEQ ID No. 38);
5'-AAACAGGCGAUUACGGAGC (antisense strand), (SEQ ID No. 39)] or GFP control siRNA (Eurogentec, Seraing, Belgium) using jetSI-ENDO (Eurogentec) following the standard procedure. On day 5, after 24 hours of starving in serum-free medium, cells were stimulated with 10 nM human recombinant renin (mammalian expression; kind gift from Dominik N. Muller, MDC Berlin) or vehicle (0.1% DMSO in PBS) for 2 hours. Western blot was performed with anti-c-myc antibody (mouse monoclonal, 9B11; 1:2,000; Cell Signalling) and horseradish peroxidase-conjugated anti-mouse antibody (rabbit polyclonal; 1:2,500; DAKO). Chemoluminescence was detected with ECL (Amersham, München, Germany), and subsequent exposition to Hyperfilm (Amersham).

*Fluorescence microscopy*

HeLa-S3 cells were seeded in 8-well chamber slides (BD Biosciences, Erembodegem, Belgium) at day 1 and transfected the following day at 30-50% confluency with 50 ng of the indicated plasmid using GeneJuice (Merck Biosciences) following the standard protocol.

For immunofluorescene microscopy, 48 h after transfection cells were fixed and permeabilized in methanol for 10 minutes at -20 °C. Blocking was performed in 1x TBS with 0.1% Tween 20, 5% skimmed milk powder and 1% BSA (Sigma-Aldrich, Taufkirchen, Germany) for 1 hour at room temperature. The primary antibodies anti-FLAG-M2 (mouse monoclonal; 1 μg/ ml; Sigma-Aldrich, Taufkirchen, Germany) and anti-c-myc-tag (mouse monoclonal, 9B11; 1:2,000; Cell Signalling) were incubated overnight in blocking buffer, followed by 1 hour of incubation at room temperature with Cy3- or Cy5-conjugated anti-mouse antibody (rabbit polyclonal; 1:250; DAKO) in blocking buffer. DAPI (Invitrogen, Karlsruhe, Germany) was used for nuclear staining according to the manufacturer's protocol.

For microscopy of the EGFP constructs, cells were stained with 1 μM ER-Tracker Red or 0.1 μM LysoTracker Red (both Invitrogen) in OPTIMEM (Gibco, Karlsruhe, Germany) for 30 minutes at 37 °C 48 hours after transfection and subjected to imaging.

All images were obtained with a Leica DM-IRE2 (Leica, Wetzlar, Germany) fluorescence microscope with a 63x lens.

*Yeast two-hybrid screening*

The complete coding sequence (CDS) of the human RER, based on GenBank GI:21325928, was cloned into the yeast two-hybrid bait-vector pBTM117c (kind gift from Erich Wanker, MDC, Berlin) expressing a LexA DNA binding domain using the following primers:

5'-CATGGCTGTGTTTGTCGTGCTCCT (sense primer), (SEQ ID No. 40);
5'-TCAATCCATTCGAATCTTCTGG (antisense primer), (SEQ ID No. 41).

This construct was co-transformed into L40 yeast with a GAL4 activation domain fusion library of adult human heart cDNA in pACT2 vector (Clontech, Heidelberg, Germany). Yeast two-hybrid screening was carried out according to the cDNA library manufacturer's guidelines. Positive clones were identified and checked after re-transformation on minimal medium lacking tryptophan, leucine and histidine and an additional beta-galactosidase assay.

*Co-immunoprecipitation (coIP)*

HEK293 cells were seeded on day 1 in 175 $cm^2$ flasks. Transfection was performed on day 2 using polyethylenimine at 60-80% confluency. 20 μg of each indicated plasmid were diluted in 500 μl PBS; 120 μl of a polyethylenimine solution (0.9 mg/ ml in $ddH_2O$; average MW 750,000; Sigma-Aldrich) were diluted in 500 μl 0.1 M NaCl. Both solutions were vortexed and incubated separately for 10 min at room temperature. The solutions were then mixed and again incubated for 10 min at room temperature before addition to the serum- and antibiotics-free growth medium, which was replaced with complete growth medium after 4 hours.

48 hours post-transfection, the cells were washed twice with ice-cold PBS and lysed in ice-cold lysis buffer containing 50 mM Tris-HCl (pH 7.4), 150 mM NaCl, 1 mM EDTA, 1 % Nonidet P40, 15% glycerol and Complete protease inhibitor cocktail (Roche, Mannheim, Germany). Lysate containing 2 mg of total protein (determined by the BCA method; Bio-Rad) was treated with anti-FLAG-M2-agarose affinity beads, EZview Red anti-c-Myc Affinity Gel or protein A agarose only (all Sigma-Aldrich), respectively, according to the manufacturer's recommendations. 25% of eluate were subjected to a 10% SDS-PAGE and transferred to a nitrocellulose membrane. Western blot was performed with anti-FLAG-M2 antibody (mouse monoclonal; 0.5 μg/ ml; Sigma-Aldrich), anti-c-myc-tag antibody (mouse monoclonal, 9B 11; 1:2,000; Cell Signalling) and horseradish peroxidase-conjugated anti-mouse antibody (rabbit polyclonal; 1:2,500; DAKO, Hamburg). Detection was performed as described above.

For endogenous coIP, total lysates were prepared as described above and subjected to immunoprecipitation using an anti-PLZF antibody (rabbit polyclonal, sc-22839; Santa Cruz Biotechnology) non-covalently bound to protein A agarose or protein A agarose only. Western blot was performed as described above with anti-PLZF antibody (rabbit polyclonal, sc-22839; Santa Cruz Biotechnology) and anti-RER antibody (goat polyclonal, ab5959, Abcam, Cambridge, UK), respectively.

*Luciferase assay and real-time RT-PCR under siRNA and renin stimulation conditions*

HEK293 cells were seeded in 24-well plates and transfected with either 100 ng of pCEP4-PLZF-myc expression vector, 50 ng of indicated pGL3-basic construct and 10 ng of phRL-null plasmid (for luciferase assay), or 100 ng of pCEP4-PLZF-myc alone (for PCR experiments) per well as described above. siRNA transfection, renin stimulation and determination of reporter activity were performed as described above, except for an exceeded renin stimulation period of 3 hours.

*Quantitative real-time RT-PCR (qPCR)*

RNA isolation and cDNA synthesis were performed as described above. qPCR was performed applying a SYBR Green I reaction mix and run on a Stratagene Mx3000P (Stratagene, La Jolla, CA, USA) using the following primers:

human RER: 5'-AAACAAGCGAAGAACCCAGC (sense), (SEQ ID No. 42),
5'-GGTGATAATCACAGCCAAGGC (antisense), (SEQ ID No. 43);
human PI3K-p85α: 5'-CGGATCTTGCAGAGCAGTTT (sense), (SEQ ID No. 44),
5'-AGGTTGCTGGAGCTCTGTGT (antisense), (SEQ ID No. 45);
human PLZF: 5'-TAGGGTGCACACAGGTGAGA (sense), (SEQ ID No. 46),
5'-GTGCAGATGGTGCACTGGTA (antisense), (SEQ ID No. 47);
human 18S rRNA: 5'- CCGCAGCTAGGAATAATGGAATA (sense),
(SEQ ID No. 48),
5'-TCTAGCGGCGCAATACGAAT (antisense), (SEQ ID No. 49).

Data represents the mean expression level ± standard deviation (standardized to 18S rRNA expression) calculated according to the ddCT method of at least three independent measurements. Equality of PCR efficiencies has been verified applying the program LinRegPCR (Ramakers *et al.* 2003) and ANOVA testing.

*Electromobility shift assay (EMSA)*

EMSA experiments were performed as described previously (Funke-Kaiser *et al.,* 2003b) using the oligonucleotides 5'-CttaactacagttttcacTGG (RER native), (SEQ ID No. 50),
5'-ctttacatctgttttcactgg (RER mutated), (SEQ ID No. 51), and
5'-TTACATGTACTAGTGTTGTGG (PI3K-p85α), (SEQ ID No. 52); the binding reaction
was performed according to Senbonmatsu *et al.* (Senbonmatsu *et al.,* 2003). For super-shift analysis, an anti-PLZF antibody (rabbit polyclonal, sc-22839; Santa Cruz Biotechnology) was used; an anti-MMP2 antibody (rabbit polyclonal, sc-10736; Santa Cruz Biotechnology) served as control.

*Chromatin-immunoprecipitation (ChIP)*

HEK293 cells cultured in 75 $cm^2$ flasks were starved and stimulated with renin for 2 hours as described above. Fixation at a confluence of about 90% was performed using 1% formaldehyde in lx PBS for 7 min at room temperature. Cells were then rinsed twice with ice-cold lx PBS, abraded in lx PBS, and centrifuged for 5 min at 440 x g. Each pellet was resuspended in 1 ml lysis buffer (1% SDS; 50 mM Tris-HCI; 1x Complete protease inhibitor cocktail, Roche; 5 mM EDTA, final concentration; pH 8.1), followed by a 20 min incubation on ice. Sonification, immunoprecipitation and reversal of crosslink were performed according to Bryant and Ptashne (Bryant and Ptashne, 2003), using 3 μg of following antibodies (all from Santa Cruz Biotechnology): PLZF (sc-22839), TBP (sc-204) and MMP-2 (sc-10736). Sonification itself was performed using the Sonoplus HD 2070/UW 2070 sonifier with the tip MS 72 (Bandelin Electronic, Berlin, Germany; output control (power %) = 100; time = 20 sec (once); constant duty cycle).
qPCR was performed as described above applying the following primers:

human RER promoter around the PLZF consensus sequence:
5'-gctctgtgcctcctctctca (sense), (SEQ ID No. 53),
5'-cccagctgatgaccttgaa (antisense), (SEQ ID No. 54);
human β-actin promoter: 5'-aatgctgcactgtgcggcga (sense), (SEQ ID No. 55),
5'-ggcggatcggcaaaggcga (antisense), (SEQ ID No. 56);
human intergenic region: 5'-AAATGAAGTGAGACCCCTCC (sense),
(SEQ ID No. 57),
5'-TCAAAGGACACACAGCACCT (antisense), (SEQ ID No. 58).

Input (total) and genomic DNA served as positive controls. DNA fragments immunoprecipitated with the anti-MMP-2 antibody, as well as PCRs on an intergenic region and a no template control (NTC) served as negative controls.
Calculation of transcription factor (PLZF) recruitment (to human RER promoter) standardized to the recruitment of TBP to the TATA box region of the human beta-actin promoter (X) was calculated according to

$$X = \frac{2^{-[Ct[IP(PLZF),PCR(RER)]-Ct[input,PCR(RER)]]}}{2^{-[Ct[IP(TBP),PCR(\beta-actin)]-Ct[input,PCR(\beta-actin)]]}}$$

X was calculated separately for renin stimulation [X(renin)] and vehicle control [X(vehicle)]. X(renin) divided by X(vehicle) served as a measure for relative PLZF recruitment. Indicated standard deviation is based on three independent measurements on each template.

Concerning the negative controls, following criteria were fulfilled (">" indicates a CT difference of at least 3):

(1) CT[IP(MMP-2), PCR(RER)] > CT[IP(PLZF), PCR(RER)];
(2) CT[IP(MMP-2), PCR(β-actin)] > CT[IP(TBP), PCR(β-actin)];
(3) CT[IP(PLZF), PCR(intergenic)] - CT[total, PCR(intergenic)] > CT[IP(PLZF), PCR(RER)] - CT[total, PCR(RER)];
(4) CT[IP(TBP), PCR(intergenic)] - CT[total, PCR(intergenic)] > CT[IP(TBP), PCR(β-actin)] - CT[total, PCR(β-actin)];
(5) no template controls (NTCs) yielded no amplicons after 40 cycles.

*Statistical analysis*

A two-tailed t-test has been applied and statistical significance was assumed at $p < 0.05$.

<u>EXAMPLE 2:</u> Tissue and subcellular distribution of the RER

*mRNA expression and basal promoter analysis of human RER*

To analyse the expression pattern of the RER and to find appropriate cell lines for studying RER function, we performed a RT-PCR expression analysis of several different human neuronal, glial, epithelial and endothelial cell lines as well as human kidney and human heart (as positive controls for expression of RAS components). This analysis showed a ubiquitous mRNA expression pattern of the RER in contrast to other components of the RAS (Figure 1A). In addition, we performed a Northern blot to assess the level of RER mRNA expression. RER mRNA can be easily detected by this method indicating strong expression of this gene (Figure 1B).

To analyse the gene regulatory mechanisms responsible for the observed ubiquitous expression of the RER, we determined its transcriptional start sites and examined its promoter function. A RNA ligase-mediated-5'-RACE experiment, which only amplified capped mRNA, indicated multiple transcriptional start sites at positions 76, 86, 96, 102, 105, 112 and 125 bp upstream of the translational start codon (Figure 2A) which is consistent with the TATA box-less architecture of this promoter (Heinemeyer *et al.,* 1999). In addition, three serial deletion mutants of the human RER promoter (spanning 126, 500 and 1100 bp upstream of the translational start codon) were subcloned for luciferase reporter gene assay. The RER promoter is characterized by a very high promoter activity in the examined neuronal, endothelial and epithelial cell lines (Figure 2B). Its activity is even higher than the activity of the human ECE-1c promoter, which was shown to be a strong, ubiquitous housekeeping promoter (Figure 2B) (Funke-Kaiser *et al.,* 2000; Funke-Kaiser *et al.,* 2003c). Additionally, our results indicate that 500 bp of the human RER promoter are sufficient to drive maximal promoter activity in neuronal and cardiovascular relevant cell types (Figure 2B).

*Subcellular localization of the RER*

Since the subcellular localization of the RER may be a clue for the understanding of its function, we addressed this question using different methods. Initially, the localization of the RER within cellular membranes (i.e., plasma membrane and/or membranes of organelles) was demonstrated by fractionated protein isolation followed by Western blotting of HeLa-S3 cells (Figure 3A). The observed molecular weight of about 38-39 kDa in the membrane fraction is consistent with the size of full-length RER described by Nguyen and colleagues. (Nguyen *et al.,* 2002), whereas the lower band of about 35-36 kDa seen in cytosolic and membrane fractions (Figure 3A) likely corresponds to the Δ4-splice variant reported by Ramser and colleagues (Ramser *et al.,* 2005) in which the 96 bp-sized exon 4 is missing. To further analyse the cellular membrane compartment, in which the RER is localized, we performed a set of fluorescence microscopy experiments. Immunocytology of c-myc- and FLAG-tagged RER in HeLa-S3 cells indicated a perinuclear localization (Figure 3B). We next generated three dif ferent EGFP fusion proteins of the RER (each as N- and C-terminal fusion): (1) a full-length wild type RER (RER full), (2) a full-length RER, in which its atypical endoplasmic reticulum (ER)-retention motif KXXXX (Vincent *et al.,* 1998; Gaynor *et al.,* 1994) was mutated to RXXXX (RER K/R mut), and (3) the V-ATPase segment of the RER (RER ATPase). The full-length RER construct showed again a perinuclear localization and colocalized with a marker of the ER (Figure 3C). Mutagenesis of the ER retention motif resulted in a loss of perinuclear/ER localization (Figure 3C). Interestingly, the V-ATPase segment of the RER showed a different localization pattern compared to the full-length RER as it was localized primarily to the lysosomal compartment (Figure 3C). EGFP N-terminally fused to these constructs yielded similar results (data not shown).

The primary perinuclear subcellular localization of the RER observed in our work conflicts with the plasma membrane localization described by Nguyen and colleagues (Nguyen *et al.,* 2002). Nevertheless, all our experiments - including use of different constructs, mutagenesis, and co-localization studies - indicated an intracellular localization of the RER. Furthermore, the total cellular membrane lysates used for their kinetic studies may still contain intracellular membrane proteins, and, therefore, do not exclude an intracellular localization of the RER. The possibility that our results concerning the perinuclear localization are caused by an artefact related to tags or overexpression is unlikely since we used several different C-terminal and also N-terminal tags. In addition, mutagenesis of the atypical C-terminal ER-retention signal strongly reduced the perinuclear localization of the RER. The fact that CAPER, which is identical to the RER as discussed above, can directly bind to PRL-1 - a protein observed in the ER in non-mitotic cells - also supports our results. PRL-1 is involved in the regulation of cellular proliferation and transformation, and exhibits a cell cycle-dependent subcellular localization, being localized to the endoplasmic reticulum (ER) in resting cells and to centrosomes and the spindle apparatus in mitotic cells (Wang *et al.,* 2002).

On the other hand, our experiments indicate that an extracellular signal (renin) can affect the signal transduction of the RER despite its mainly intracellular localization. Several mechanisms might account for this observation. First, other renin-binding receptors, such as the mannose-6-phosphate receptor, could internalize renin and prorenin (Danser and Deinum, 2005). Secondly, a nonsecreted (i.e., intracellular) renin isoform, which could directly interact with an intracellular RER (see below), has been described containing an alternative first exon termed - albeit identical - "exon 1b" (Lee-Kirsch *et al.,* 1999; Sinn and Sigmund, 2000) and "exon 1A" (Clausmeyer *et al.,* 1999; Peters and Clausmeyer, 2002), respectively. Thirdly, it could be possible that very small amounts of the RER within the plasma membrane are sufficient for the initiation of a RER signal transduction cascade. In this context, the observed homodimerization of the RER might also be of importance, since dimerization can affect subcellular localization (Muller *et al.,* 2003).

In this study, we were able to demonstrate a ubiquitous expression of the human RER, a high promoter activity in different cell types and multiple transcriptional start sites in a TATA boxless promoter. These features are consistent with housekeeping properties of the RER gene (Funke-Kaiser *et al.,* 2003c), suggesting basal cellular functions of this protein. In this context it is of interest that the C-terminal part of the RER is - as discussed above - identical to the vacuolar proton-translocating ATPase (V-ATPase) membrane sector-associated protein M8-9, since V-ATPase have functions in almost every eukaryotic cell (Nelson and Harvey, 1999). Nevertheless, APT6M8-9 only constitutes 69 to 100 amino acids (GenBank identifier number GI:5031590; Ludwig *et al.,* 1998) of the 350 amino acids (Nguyen *et al.,* 2002) of the full-length renin receptor. Therefore, the functions of the RER exceed those of a pure V-ATPase subunit which is also supported by the data on the signal transduction and the different subcellular localizations of full-length RER and APT6M8-9 provided here. Alternative promoters and posttranslational cleavage by an unidentified protease are putative explanations.

<u>EXAMPLE 3:</u> Protein-protein interaction partners of the RER

No direct molecular interactions of the RER have been described so far. Therefore, a major objective of our study was to identify protein interaction partners of this ubiquitously expressed receptor to gain insight into its signal transduction cascade. For this purpose, we performed a yeast two-hybrid screening using a human adult heart cDNA library (prey) and the full-length human RER (bait). The C-terminal third of the transcription factor PLZF was identified as RER interacting protein in four clones, three of which were independent. To confirm this RER-PLZF interaction, we performed a co-immunoprecipitation (coIP) utilizing transient transfections of full-length human RER and full-length human PLZF. Figure 4A demonstrates the ability of the RER to interact with PLZF in a system using tagged proteins. This finding was further confirmed in an endogenous context (Figure 4B).

In this work we identified the transcription factor PLZF as the first direct protein-protein interaction partner of the human RER. Furthermore, we identified the cytoplasmatic C-terminal tail of the RER as domain interacting with PLZF (Figure 4C).

Since dimerization is a common feature of several receptors, we investigated if homodimerization may also be a characteristic of the RER. For this purpose human RER constructs with two different tags were transiently transfected. CoIP experiments indicated that the RER is able to form homodimers. (Figure 4D).

The transcription factor PLZF contains multiple zink-finger domains and is disrupted in patients with acute promyelocytic leukemia (APL) caused by t(11;17)(q23;q21) chromosomal translocation (Costoya and Pandolfi, 2001; Minucci and Pelicci, 2006). This APL subform is characterized by PLZF-RAR$\alpha$ (retinoic acid receptor-alpha) fusion proteins, which recruit histone deacetylase 1 (HDAC1) and which do not respond to retinoic acid (RA) any more, explaining the missing response of these patients to RA treatment (Minucci and Pelicci, 2006; Grignani *et al.,* 1998; Glaser *et al.,* 2003).

Wild type PLZF can act as growth repressor and exerts pro-apoptotic functions during development (Costoya and Pandolfi, 2001; Barna *et al.,* 2000). Concerning the RAS, it seems important to note that PLZF was recently described as an adaptor protein of the angiotensin AT2 receptor (AT2R) in the heart (Senbonmatsu *et al.,* 2003). This direct PLZF-AT2R interaction was associated with stimulation of protein synthesis and putative cardiac hypertrophy (Senbonmatsu *et al.,* 2003).

Data obtained from PLZF knockout mice indicate that this transcription factor is involved in limb and axial skeletal patterning (Barna *et al.,* 2000), whereas the brain phenotype of these mice was not described by the authors. Consistent with this observation, PLZF target genes include hox genes (Barna *et al.,* 2000; Ivins *et al.,* 2003; Barna *et al.,* 2002), besides the p85α subunit of PI3K (Senbonmatsu *et al.,* 2003) and cyclin A2 (Yeyati *et al.,* 1999).

EXAMPLE 4: Signal transduction downstream of the RER

*Functional analysis of the renin-RER-PLZF signal transduction pathway*

In initial experiments involving PLZF overexpression in HEK293 cells (which endogenously express RER and PLZF mRNA (data not shown)), we observed that RER mRNA is reduced by renin stimulation (to 80.6±3.3%) and PLZF transfection (to 72.6±5.2%), respectively (Figure 5A). A combination of both repressed RER mRNA to 45.4±4.7% (Figure 5A). We could substantiate these findings by using serial deletion mutants of the human RER promoter as described above. Only a promoter construct comprising the region [-501; -1100] can be repressed by PLZF cotransfection (Figure 5B).

Bioinformatic analysis of the RER promoter using MatInspector (http://www.genomatix.de) (Cartharius, 2005) indicated the presence of a PLZF consensus sequence (5'-aactacagttttcac) (SEQ ID No. 59) with a high core and matrix similarity located in the region [-1097; -1083] relative to the translational start codon of the promoter.

To investigate functional downstream effects of the RER-PLZF interaction and to analyse if these are influenced by stimulation of the RER with its ligand, renin, we performed a set of experiments in which the different components of the putative renin-RER-PLZF pathway and the promoter of the human RER were experimentally modulated in HEK293 cells.

RER stimulation was performed by renin incubation; dependence on RER was analysed by siRNA against this receptor (which repressed RER mRNA to 29.8±5.2%); the influence of PLZF was examined by cotransfection experiments with a PLZF expression vector (overexpression was confirmed by real-time PCR (data not shown)); and the functional importance of the PLZF consensus sequence within the human RER promoter was evaluated by site-directed mutagenesis of luciferase reporter constructs. Read-outs comprised RER promotor activity and mRNA level. The known PLZF target gene, the p85α subunit of the phosphatidylinositol-3 kinase (PI3K-p85α), served as an additional downstream candidate gene of a RER-PLZF pathway activation (Senbonmatsu *et al.,* 2003).

Stimulation of the RER with renin resulted in a decrease in the activity of a wild type RER promoter by about 30% compared to all controls (Figure 6A). Importantly, the presence of the RER was necessary for this effect (Figure 6A).

Consistent with this repressive effect of PLZF on the RER promoter, site-directed mutagenesis of the PLZF consensus sequence [-1097; -1083] caused a derepression (to over 150%) of promoter activity. The effect of renin stimulation was also abolished by this mutagenesis (Figure 6A).

Similar but inverse effects were observed with respect to the PI3K-p85α promoter which is known to be positively regulated by PLZF (Senbonmatsu *et al.,* 2003). RER stimulation with renin caused an increase of 45% in PI3K-p85α promoter activity compared to control; this effect was abolished by downregulation of the RER using siRNA (Figure 6B).

To verify these findings, we analysed the effect of RER stimulation on PI3K-p85α mRNA by real-time PCR analysis. Consistent with our promoter data, stimulation with renin increased PI3K-p85α mRNA in systems with and without PLZF overexpression by 105.2±6.74% or 30.2±7.9% (relative to vehicle control), respectively (Figure 6C).

Again, downregulation of RER by siRNA abolished this induction regardless of the expression level of PLZF as measured by mRNA expression level (Figure 6C). Additionally, it was shown that the siRNA reduced RER protein (Figure 6D). Densitometric measurement indicates a reduction to less than 10% (compared to control).

*Translocation and promoter recruitment of PLZF upon renin stimulation*

Finally, we examined, whether activation of the RER by renin is able to cause a translocation of PLZF from the cytoplasm to the nucleus. The subcellular localization of c-myc-tagged PLZF was evaluated by Western blotting after fractionated extraction of nuclear and cytosolic proteins. Incubation of HEK293 cells with renin caused a clear increase in nuclear PLZF, whereas cytoplasmic PLZF almost disappeared (Figure 7A). Importantly, this translocation of PLZF again required the presence of the RER as indicated by our siRNA experiments (Figure 7A).

The additional experiment underlying Figure 7B further substantiates the translocation of PLZF into the nucleus upon RER stimulation by renin under native condition.

To evaluate the recruitment of endogenous PLZF to the human RER promoter within the chromatin context, we performed a chromatin-immunoprecipitation (ChIP) experiment. To ensure valid quantification of transcription factor recruitment, immunoprecipitated DNA was quantified applying real-time PCR analysis and multiple positive and negative controls. Renin stimulation of HEK293 cells did increase the PLZF recruitment to the RER promoter region encompassing the PLZF cis-element at position [-1097; -1083] about 6-fold (Figure 7C).

The binding of PLZF to this cis-element of the human RER promoter - and not to a mutated form - was further confirmed by EMSA (Figure 7D), since we found a positive super-shifted band using a PLZF antibody. Furthermore, the observed pattern was identical compared to the positive control oligonucleotide derived from the PI3K-p85α promoter (Senbonmatsu *et al.,* 2003).

EXAMPLE 5: Construction of the RER signal transduction pathway

Herein, we describe a novel, short-loop signal transduction pathway involving renin, RER, the transcription factor PLZF and two direct downstream targets, PI3K-p85α and the RER itself (Figure 8).

Stimulation of the RER with its ligand renin causes a translocation of PLZF from the cytoplasm to the nucleus and a recruitment of PLZF to promoter regions of PI3K-p85α and the RER. Depending on the promoter context, PLZF is able to activate the transcription of PI3K-p85α and to repress the gene expression of the RER. This dual role of PLZF as activator (Labbaye *et al.,* 2002; Senbonmatsu *et al.,* 2003) and also repressor (Li *et al.,* 1997; Yeyati *et al*., 1999) has been described before, but to our knowledge not in a single cellular context, and, in particular, not in connection with RER signal transduction. The fascinating and uncommon observation that a receptor directly interacts with a transcription factor speaks for an extraordinary short signal transduction cascade. Similar mechanisms, by which a transcription factor or nuclear factor is directly activated at a receptor site or at an extranuclear membrane, have been described for the SMAD, JAK-STAT, Notch and SREBP pathways (Brivanlou and Darnell, 2002; Emery *et al.,* 2001). Remarkably, PLZF itself represses the promoter of its own direct interaction partner RER, thereby establishing a very short negative feedback loop. The observation that a RER promoter with a mutated PLZF cis-element is refractory to effects of renin stimulation (Figure 6A) indicates that this DNA motif is necessary for this feedback mechanism. Binding of PLZF to this motif was further confirmed by our EMSA experiments (Figure 7C). Most importantly, the recruitment of native PLZF to the corresponding cis-element region of the RER promoter was demonstrated in the chromatin context by ChIP, with a significant increase under renin stimulation (Figure 7B). The ability of renin to decrease the gene expression of RER and to increase the gene expression of PI3K-p85α depends on the presence of the RER. Therefore, our data further prove the existence of the RER as a functional renin receptor, thereby confirming the work of Nguyen and colleagues (Nguyen *et al.,* 2002).

**References**

Amsterdam A, Nissen RM, Sun Z, Swindell EC, Farrington S and Hopkins N (2004) Identification of 315 genes essential for early zebrafish development. Proc Natl Acad Sci USA 101: 12792-12797

Avantaggiato V, Pandolfi PP, Ruthardt M, Hawe N, Acampora D, Pelicci PG and Simeone A (1995) Developmental analysis of murine Promyelocyte Leukemia Zinc Finger (PLZF) gene expression: implications for the neuromeric model of the forebrain organization. J Neurosci 15: 4927-4942

Barna M, Hawe N, Niswander L and Pandolfi PP (2000) Plzf regulates limb and axial skeletal patterning. Nat Genet 25: 166-172

Barna M, Merghoub T, Costoya JA, Ruggero D, Branford M, Bergia A, Samori B and Pandolfi PP (2002) Plzf mediates transcriptional repression of HoxD gene expression through chromatin remodeling. Dev Cell 3: 499-510

Brivanlou AH and Darnell JE, Jr. (2002) Signal transduction and the control of gene expression. Science 295: 813-818

Bryant GO and Ptashne M (2003) Independent recruitment in vivo by Gal4 of two complexes required for transcription. Mol Cell 11: 1301-1309

Burckle CA, Jan Danser AH, Muller DN, Garrelds IM, Gasc JM, Popova E, Plehm R, Peters J, Bader M and Nguyen G (2006) Elevated blood pressure and heart rate in human renin receptor transgenic rats. Hypertension 47: 552-556

Cartharius K, Frech K, Grote K, Klocke B, Haltmeier M, Klingenhoff A, Frisch M, Bayerlein M and Werner T (2005) MatInspector and beyond: promoter analysis based on transcription factor binding sites. Bioinformatics 21: 2933-2942

Clausmeyer S, Sturzebecher R and Peters J (1999) An alternative transcript of the rat renin gene can result in a truncated prorenin that is transported into adrenal mitochondria. Circ Res 84: 337-344

Costoya JA and Pandolfi PP (2001) The role of promyelocytic leukemia zinc finger and promyelocytic leukemia in leukemogenesis and development. Curr Opin Hematol 8: 212-217

Danser AH and Deinum J (2005) Renin, prorenin and the putative (pro)renin receptor. Hypertension 46: 1069-1076

Emery JG, Ohlstein EH and Jaye M (2001) Therapeutic modulation of transcription factor activity. Trends Pharmacol Sci 22: 233-240

Funke-Kaiser H, Bolbrinker J, Theis S, Lemmer J, Richter CM, Paul M and Orzechowski HD (2000) Characterization of the c-specific promoter of the gene encoding human endothelin-converting enzyme-1 (ECE-1). FEBS Lett 466:

310-316
Funke-Kaiser H, Lemmer J, Langsdorff CV, Thomas A, Kovacevic SD, Strasdat M, Behrouzi T, Zollmann FS, Paul M and Orzechowski HD (2003a) Endothelin-converting enzyme-1 (ECE-1) is a downstream target of the homeobox transcription factor Nkx2-5. Faseb J 17: 1487-1489
Funke-Kaiser H, Reichenberger F, Kopke K, Herrmann SM, Pfeifer J, Orzechowski HD, Zi- dek W, Paul M and Brand E (2003b) Differential binding of transcription factor E2F-2 to the endothelin-converting enzyme-1b promoter affects blood pressure regulation. Hum Mol Genet 12: 423-433
Funke-Kaiser H, Thomas A, Bremer J, Kovacevic SD, Scheuch K, Bolbrinker J, Theis S, Lemmer J, Zimmermann A, Zollmann FS, Herrmann SM, Paul M and Orzechowski HD (2003c) Regulation of the major isoform of human endothelin-converting enzyme-1 by a strong housekeeping promoter modulated by polymorphic microsatellites. J Hypertens 21: 2111-2124
Gaynor EC, te Heesen S, Graham TR, Aebi M and Emr SD (1994) Signal-mediated retrieval of a membrane protein from the Golgi to the ER in yeast. J Cell Biol 127: 653-665
Glaser KB, Staver MJ, Waring JF, Stender J, Ulrich RG and Davidsen SK (2003) Gene expression profiling of multiple histone deacetylase (HDAC) inhibitors: defining a common gene set produced by HDAC inhibition in T24 and MDA carcinoma cell lines. Mol Cancer Ther 2: 151-163
Grignani F, De Matteis S, Nervi C, Tomassoni L, Gelmetti V, Cioce M, Fanelli M, Ruthardt M, Ferrara FF, Zamir I, Seiser C, Grignani F, Lazar MA, Minucci S and Pelicci PG (1998) Fusion proteins of the retinoic acid receptor-alpha recruit histone deacetylase in promyelocytic leukaemia. Nature 391: 815-818
Hershey JC, Steiner, B., Fischli, W. and Feuerstein, G. (2005) Renin inhibitors: An antihypertensive strategy on the verge of reality. Drug Discovery Today: Therapeutic Strategies 2: 181-185
Ichihara A, Hayashi M, Kaneshiro Y, Suzuki F, Nakagawa T, Tada Y, Koura Y, Nishiyama A, Okada H, Uddin MN, Nabi AH, Ishida Y, Inagami T and Saruta T (2004) Inhibition of diabetic nephropathy by a decoy peptide corresponding to the "handle" region for nonproteolytic activation of prorenin. J Clin Invest 114: 1128-1135
Ichihara A, Kaneshiro, Y., Takemitsu, T., Suzuki, F., Nakagawa, T., Nishiyama, A., Iwata, H., Ishida, Y., Inagami, T., Saruta, T., Hayashi, M. and. (2005) "Receptor-associated prorenin system" contributes to hypertensive end-organ damage. J Hypertens 23 (suppl 2): S78: P71.197
Ivins S, Pemberton K, Guidez F, Howell L, Krumlauf R and Zelent A (2003) Regulation of Hoxb2 by APL-associated PLZF protein. Oncogene 22: 3685-3697
Juillerat-Jeanneret L, Celerier J, Chapuis Bernasconi C, Nguyen G, Wostl W, Maerki HP, Janzer RC, Corvol P and Gasc JM (2004) Renin and angiotensinogen expression and functions in growth and apoptosis of human glioblastoma. Br J Cancer 90: 1059-1068
Labbaye C, Quaranta MT, Pagliuca A, Militi S, Licht JD, Testa U and Peschle C (2002) PLZF induces megakaryocytic development, activates Tpo receptor expression and interacts with GATA1 protein. Oncogene 21: 6669-6679
Lee-Kirsch MA, Gaudet F, Cardoso MC and Lindpaintner K (1999) Distinct renin isoforms generated by tissue-specific transcription initiation and alternative splicing. Circ Res 84: 240-246
Li JY, English MA, Ball HJ, Yeyati PL, Waxman S and Licht JD (1997) Sequence-specific DNA binding and transcriptional regulation by the promyelocytic leukemia zinc finger protein. JBiol Chem 272: 22447-22455
Ludwig J, Kerscher S, Brandt U, Pfeiffer K, Getlawi F, Apps DK and Schagger H (1998) Identification and characterization of a novel 9.2-kDa membrane sector-associated protein of vacuolar proton-ATPase from chromaffin granules. J Biol Chem 273: 10939-10947
Mazak I, Wellner, M., Shagdarsuren, E., Dechend, R., Schulze-Forster, K., Nguyen, G., Luft, F.C., Muller, D.N. (2003) Renin induces ERK 1/2 phosphorylation in U937 monocyte/macrophages independent of angiotensin II. Abstract book, 57th annual fall conference and scientific sessions of the CHBPR. Washington, DC, September, 23-26
Minucci S and Pelicci PG (2006) Histone deacetylase inhibitors and the promise of epigenetic (and more) treatments for cancer. Nat Rev Cancer 6: 38-51
Muller L, Barret A, Etienne E, Meidan R, Valdenaire O, Corvol P and Tougard C (2003) Het-erodimerization of endothelin-converting enzyme-1 isoforms regulates the subcellular distribution of this metalloprotease. J Biol Chem 278: 545-555
Nelson N and Harvey WR (1999) Vacuolar and plasma membrane proton-adenosinetriphosphatases. Physiol Rev 79: 361-385
Nguyen G, Burckle CA and Sraer JD (2004) Renin/prorenin-receptor biochemistry and functional significance. Curr Hypertens Rep 6: 129-132
Nguyen G, Delarue F, Berrou J, Rondeau E and Sraer JD (1996) Specific receptor binding of renin on human mesangial cells in culture increases plasminogen activator inhibitor-1 antigen. Kidney Int 50: 1897-1903
Nguyen G, Delarue F, Burckle C, Bouzhir L, Giller T and Sraer JD (2002) Pivotal role of the renin/prorenin receptor in angiotensin II production and cellular responses to renin. J Clin Invest 109: 1417-1427
Nussberger J, Wuerzner G, Jensen C and Brunner HR (2002) Angiotensin II suppression in humans by the orally active renin inhibitor Aliskiren (SPP100): comparison with enalapril. Hypertension 39: E1-8

Orzechowski HD, Gunther A, Menzel S, Zimmermann A, Funke-Kaiser H, Real R, Subkowski T, Zollmann FS and Paul M (2001) Transcriptional mechanism of protein kinase C-induced isoform-specific expression of the gene for endothelin-converting enzyme-1 in human endothelial cells. Mol Pharmacol 60: 1332-1342

Peters J and Clausmeyer S (2002) Intracellular sorting of renin: cell type specific differences and their consequences. J Mol Cell Cardiol 34: 1561-1568

Ramakers C, Ruijter JM, Deprez RH and Moorman AF (2003) Assumption-free analysis of quantitative real-time polymerase chain reaction (PCR) data. Neurosci Lett 339: 62-66

Ramser J, Abidi FE, Burckle CA, Lenski C, Toriello H, Wen G, Lubs HA, Engert S, Stevenson RE, Meindl A, Schwartz CE and Nguyen G (2005) A unique exonic splice enhancer mutation in a family with X-linked mental retardation and epilepsy points to a novel role of the renin receptor. Hum Mol Genet 14: 1019-1027

Re RN (2003) Intracellular renin and the nature of intracrine enzymes. Hypertension 42: 117-122

Senbonmatsu T, Saito T, Landon EJ, Watanabe O, Price E, Jr., Roberts RL, Imboden H, Fitzgerald TG, Gaffney FA and Inagami T (2003) A novel angiotensin II type 2 receptor signaling pathway: possible role in cardiac hypertrophy. Embo J22: 6471-6482

Sinn PL and Sigmund CD (2000) Identification of three human renin mRNA isoforms from alternative tissue-specific transcriptional initiation. Physiol Genomics 3: 25-31 1

Vincent MJ, Martin AS and Compans RW (1998) Function of the KKXX motif in endoplasmic reticulum retrieval of a transmembrane protein depends on the length and structure of the cytoplasmic domain. J Biol Chem 273: 950-956

Wang J, Kirby CE and Herbst R (2002) The tyrosine phosphatase PRL-1 localizes to the endoplasmic reticulum and the mitotic spindle and is required for normal mitosis. J Biol Chem 277: 46659-46668

Yeyati PL, Shaknovich R, Boterashvili S, Li J, Ball HJ, Waxman S, Nason-Burchenal K, Dmitrovsky E, Zelent A and Licht JD (1999) Leukemia translocation protein PLZF inhibits cell growth and expression of cyclin A. Oncogene 18: 925-934

## Claims

1. A method for determination of renin/prorenin receptor (RER) activity, wherein promyelocytic zinc finger protein (PLZF) activity is used as a measurement for RER activity.

2. The method according to claim 1, wherein a stimulation of RER activity is detected by RER/PLZF protein interaction and/or PLZF translocation and/or PLZF recruitment.

3. The method according to claim 2, wherein RER/PLZF protein interaction involves an interaction domain comprising the cytoplasmatic C-terminal tail of RER.

4. The method according to claim 3, wherein the interaction domain comprises an amino acid sequence encoded by a DNA sequence according to SEQ ID No. 1.

5. The method according to any of claims 2 to 4, wherein RER/PLZF protein interaction is determined by a method comprising co-immunoprecipitation (coIP), and optionally Western blot analysis.

6. The method according to claim 2, wherein PLZF translocation is detected by a decrease in cytoplasmatic PLZF protein and/or an increase in nuclear PLZF protein.

7. The method according to claim 6, wherein the decrease in cytoplasmatic PLZF protein and/or the increase in nuclear PLZF protein is determined by a method comprising fractionated extraction of cytosolic and/or nuclear proteins, and optionally Western blot analysis.

8. The method according to claim 6, wherein the decrease in cytoplasmatic PLZF protein and/or the increase in nuclear PLZF protein is determined by a method comprising a cytology or histology based procedure, preferably an immunocytology or immunohistology based procedure, and optionally immunofluorescence microscopy.

9. The method according to claim 2, wherein RER/PLZF recruitment is detected by binding of PLZF protein to a RER promoter region.

10. The method according to claim 9, wherein binding of PLZF protein to the RER promoter region involves a PLZF cis-element.

11. The method according to claim 9 or 10, wherein binding of PLZF to the RER promoter region involves a DNA sequence comprising a DNA sequence according to SEQ ID No. 2.

12. The method according to any of claims 9 to 11, wherein binding of PLZF protein to the RER promoter region is determined by a method comprising chromatin-immunoprecipitation (ChIP), and optionally real-time PCR analysis.

13. The method according to any of claims 9 to 11, wherein binding of PLZF protein to the RER promoter region is determined by a method comprising an electromobility shift assay (EMSA).

14. The method according to claim 2, wherein PLZF recruitment is detected by a decrease in RER mRNA, preferably determined by a method comprising real-time PCR, Northern blot analysis or a microarray technique.

15. The method according to claim 2, wherein PLZF recruitment is detected by a decrease in RER protein, preferably determined by a method comprising an immunology based procedure, most preferably Western blot analysis, enzyme-linked immunoabsorbent assay (ELISA), or radioimmuno assay (RIA).

16. The method according to claim 2, wherein PLZF recruitment is detected by a decrease in RER promoter activity, preferably determined by a method comprising a reporter gene assay, most preferably a luciferase reporter gene assay.

17. The method according to claim 2, wherein PLZF recruitment is detected by an increase in the p85$\alpha$ subunit of phosphatidylinositol-3 kinase (PI3K-p85$\alpha$) mRNA, pref erably determined by a method comprising real-time PCR analysis, Northern blot analysis or a microarray technique.

18. The method according to claim 2, wherein PLZF recruitment is detected by a decrease in PI3K-p85$\alpha$ protein, preferably determined by a method comprising an immunology based procedure, most preferably Western blot analysis, ELISA, or RIA.

19. The method according to claim 2, wherein PLZF recruitment is detected by an increase in PI3K-p85$\alpha$ promoter activity, preferably determined by a method comprising a reporter gene assay, most preferably a luciferase reporter gene assay.

20. A RER/PLZF protein interaction domain comprising an amino acid sequence encoded by a DNA sequence according to SEQ ID No. 1.

21. A PLZF/RER promoter interaction region comprising a DNA sequence according to SEQ ID No. 2.

22. A use of the method according to any of claims 1 to 19 for identifying a RER ligand and/or for studying the effect of a RER ligand.

**23.** The use according to claim 22, wherein the RER ligand is a RER antagonist or agonist, preferably a pharmaceutically active agent.

**24.** The use according to claim 22 or 23, wherein the RER ligand is selected from a protein, a peptide, a small molecule, a decoy or a peptide decoy.

**25.** The use according to claim 22, wherein the RER ligand is a complex comprising a renin and/or prorenin and a renin inhibitor.

**26.** A use of the method according to any of claims 1 to 19 for studying undesired renin inhibitor side-effects.

**27.** A use of the method according to any of claims 1 to 19 for screening a compound library comprising at least one putative RER ligand.

**28.** A use of an amino acid sequence encoded by a DNA sequence according to SEQ ID No. 1 or a part or a derivative thereof for determining RER activity or for studying RER signal transduction.

**29.** A use of a DNA sequence according to SEQ ID No. 1 or a part or a derivative thereof for determining RER activity or for studying RER signal transduction.

**30.** A use of a DNA sequence according to SEQ ID No. 2 or a part or a derivative thereof for determining RER activity or for studying RER signal transduction.

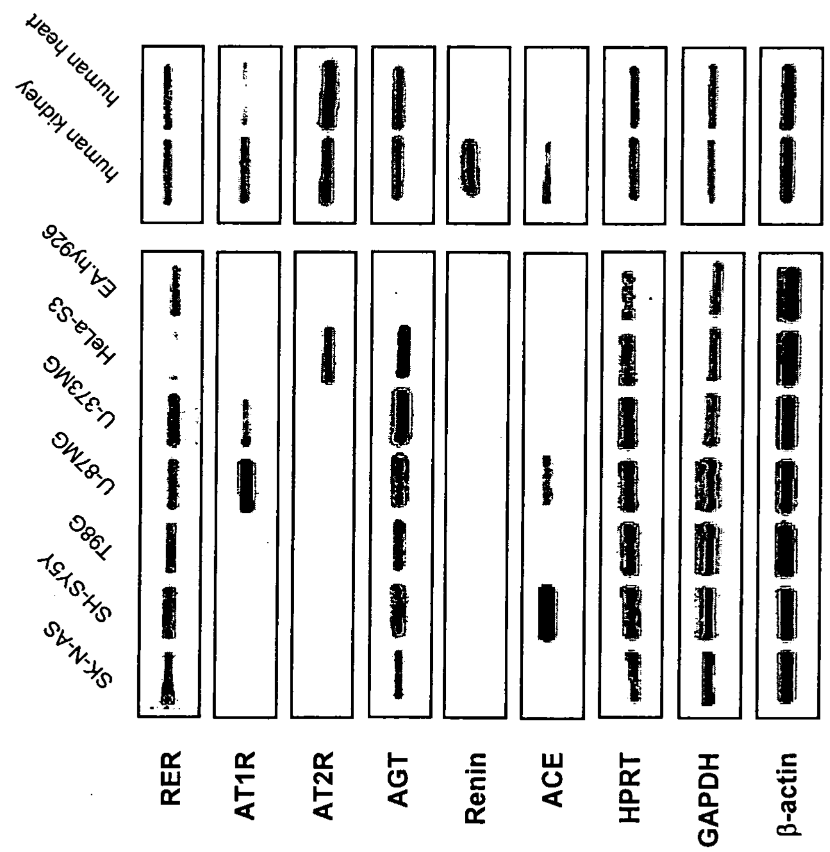
SK-N-AS
SH-SY5Y
T98G
U-87MG
U-373MG
HeLa-S3
EA.hy926
human kidney
human heart
RER
AT1R
AT2R
AGT
Renin
ACE
HPRT
GAPDH
β-actin

Fig. 1A

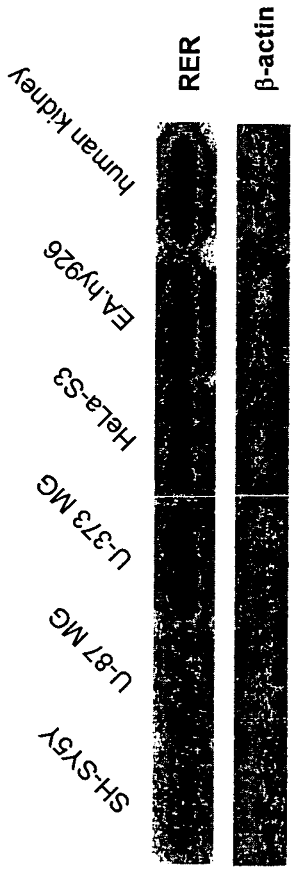
human kidney
EA.hy926
HeLa-S3
U-373 MG
U-87 MG
SH-SY5Y
RER
β-actin

Fig. 1B

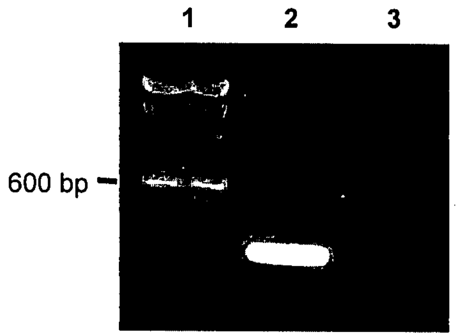


aagcggcagctgtcccagcggaagcg**a**cgaagg
gacggg**a**cccggg**a**gc**c**ggac**g**agtccgagc**g**c
gtcacctc**c**tcacgctgcggctgtcgcccgtgt
cccgccggcccgttccgtgtcgccccgcagtgc
tgcggccgccgcggcacc**atg**...

Fig. 2A

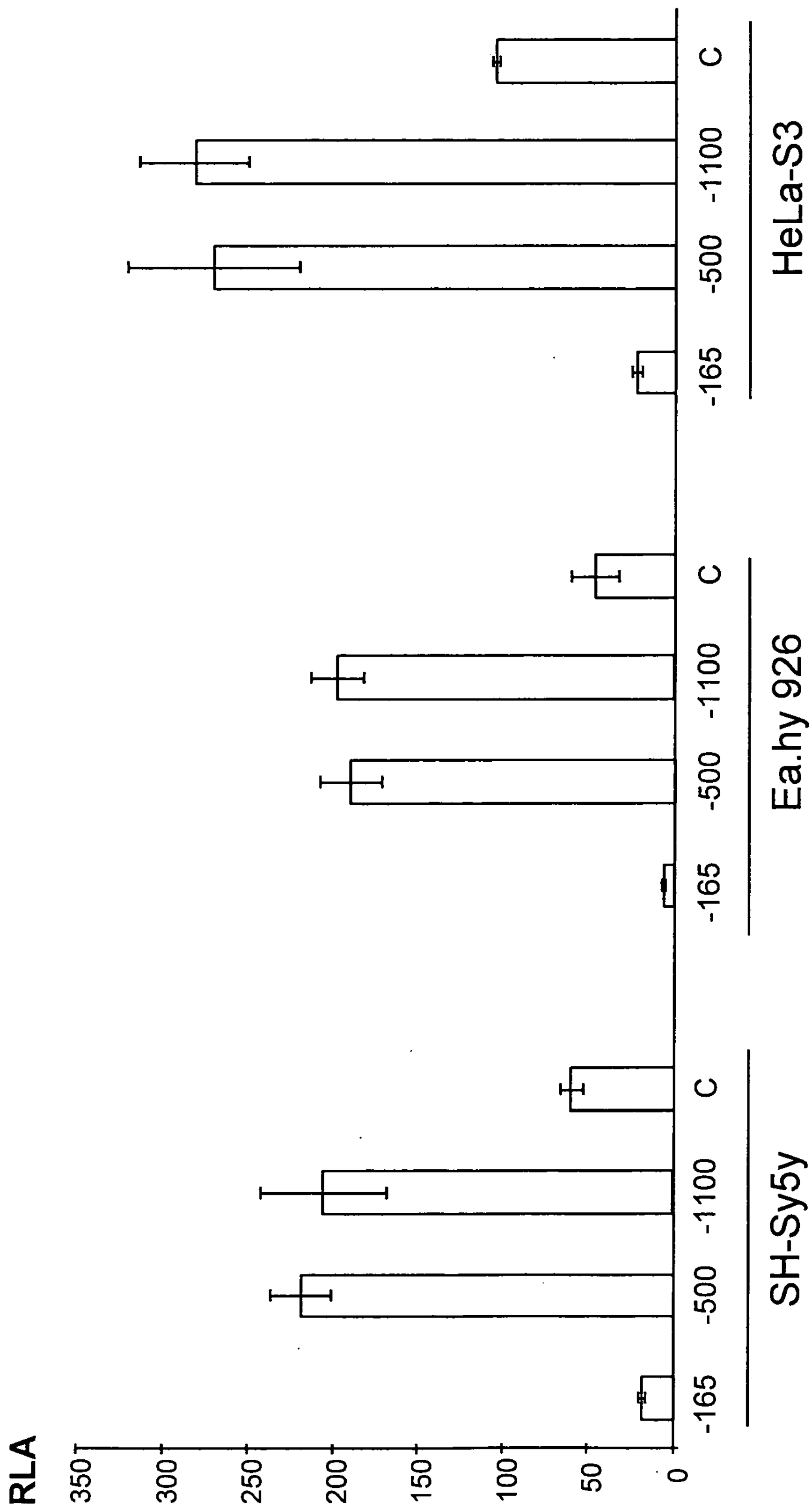
RLA
350
300
250
200
150
100
50
0
-165
-500
-1100
C
SH-Sy5y
-165
-500
-1100
C
Ea.hy 926
-165
-500
-1100
C
HeLa-S3

Fig. 2B

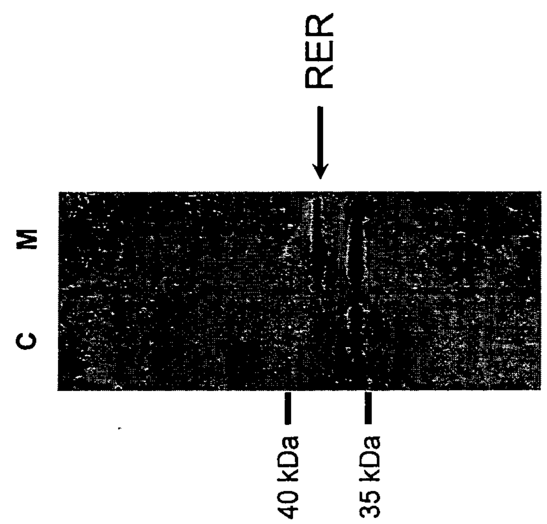


Fig. 3A

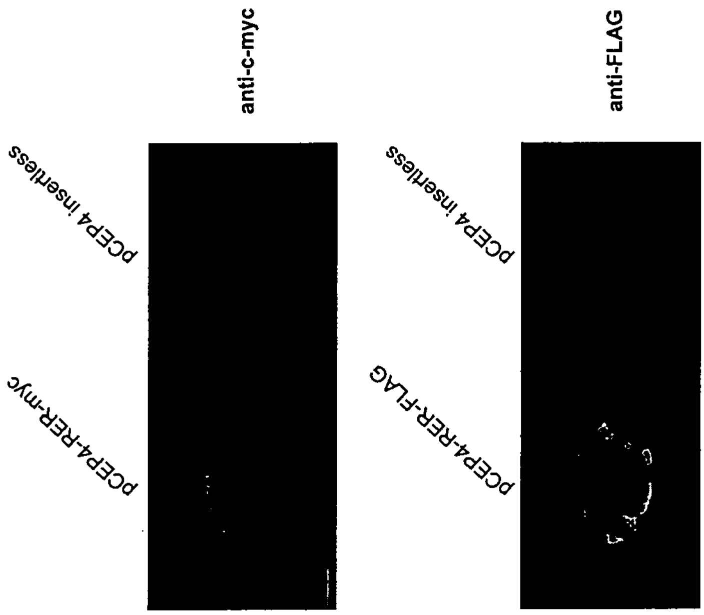
anti-c-myc
pCEP4 insertless
pCEP4-RER-myc
anti-FLAG
pCEP4 insertless
pCEP4-RER-FLAG

Fig. 3B

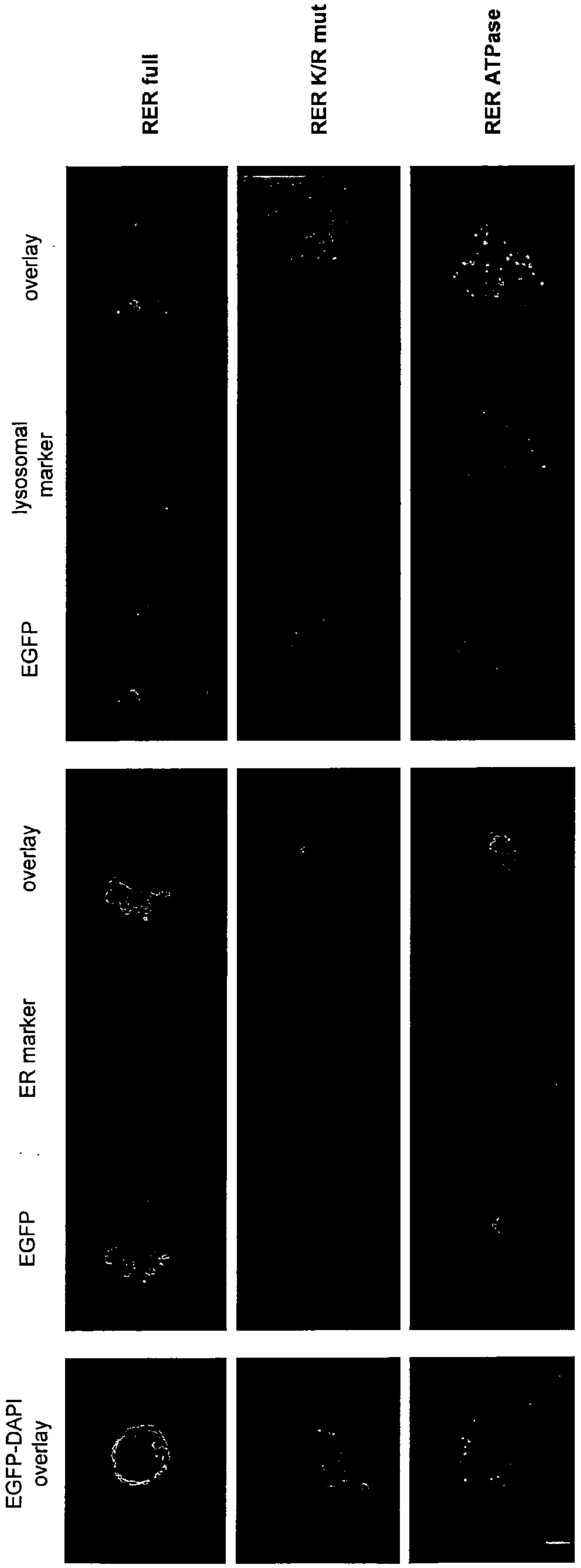
RER full
RER K/R mut
RER ATPase
overlay
lysosomal marker
EGFP
overlay
ER marker
EGFP
EGFP-DAPI overlay

Fig. 3C

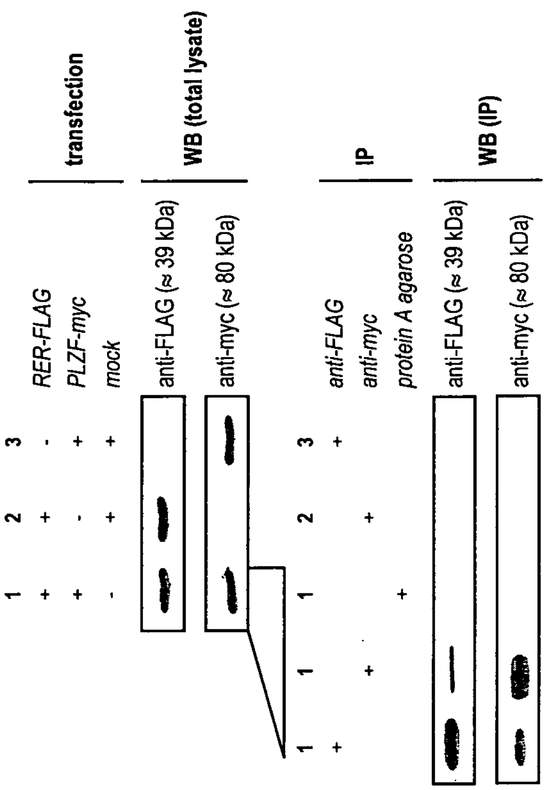
transfection
RER-FLAG
PLZF-myc
mock
WB (total lysate)
anti-FLAG (≈ 39 kDa)
anti-myc (≈ 80 kDa)
IP
anti-FLAG
anti-myc
protein A agarose
WB (IP)
anti-FLAG (≈ 39 kDa)
anti-myc (≈ 80 kDa)

Fig. 4A

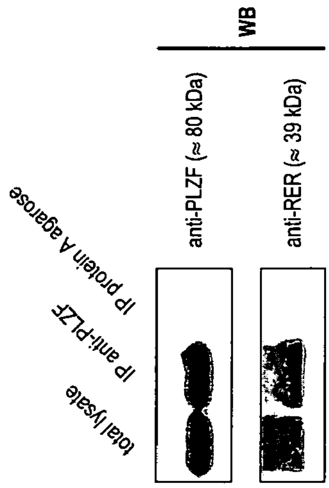
IP protein A agarose
IP anti-PLZF
total lysate
anti-PLZF (≈ 80 kDa)
anti-RER (≈ 39 kDa)
WB

Fig. 4B

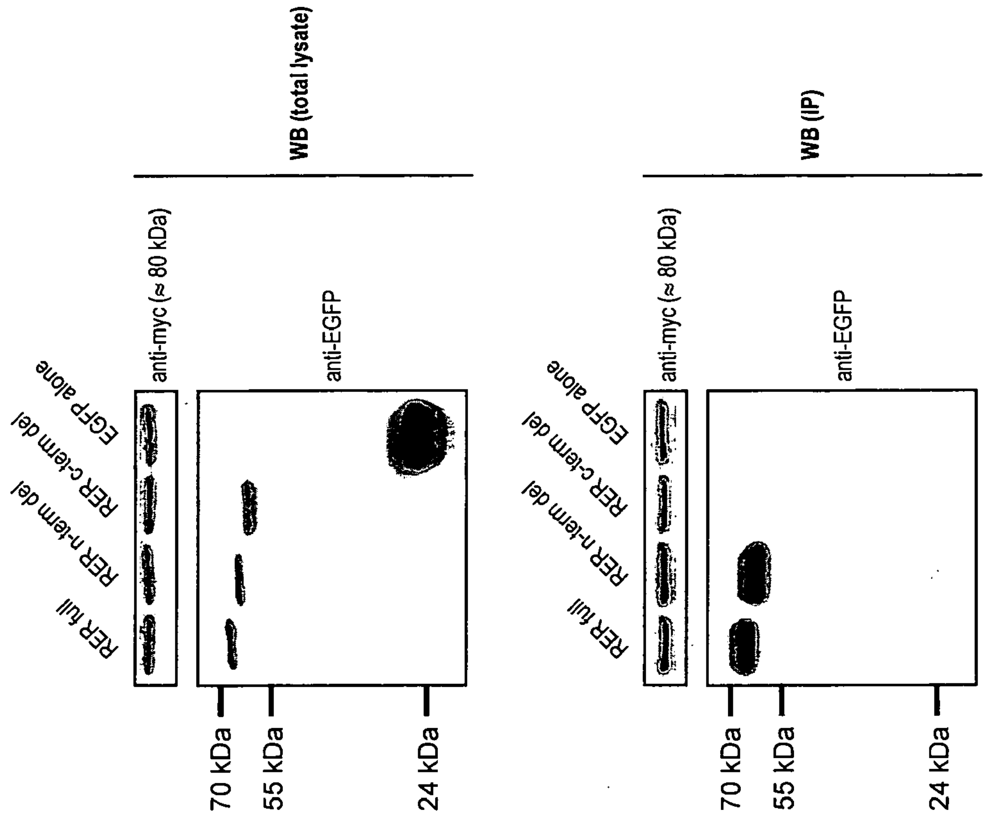
WB (total lysate)
WB (IP)
anti-myc (≈ 80 kDa)
anti-EGFP
EGFP alone
RER c-term del
RER n-term del
RER full
70 kDa
55 kDa
24 kDa

Fig. 4C

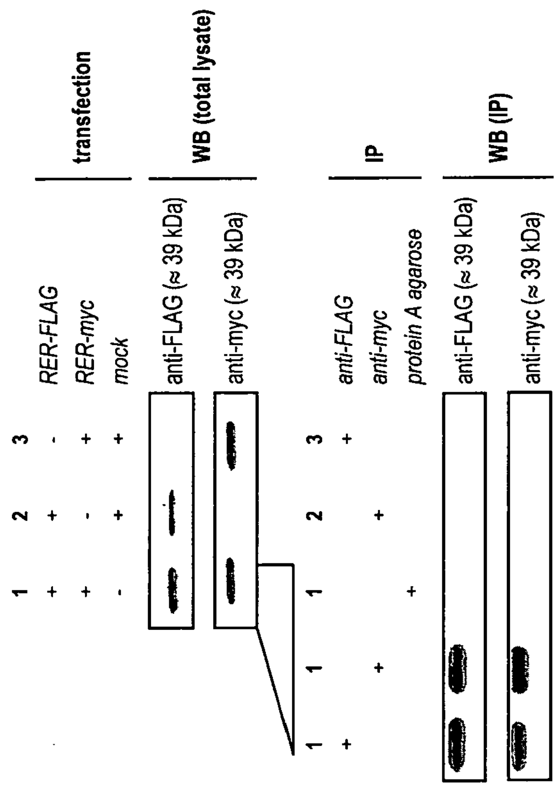
transfection
RER-FLAG
RER-myc
mock
WB (total lysate)
anti-FLAG (≈ 39 kDa)
anti-myc (≈ 39 kDa)
IP
anti-FLAG
anti-myc
protein A agarose
WB (IP)
anti-FLAG (≈ 39 kDa)
anti-myc (≈ 39 kDa)

Fig. 4D

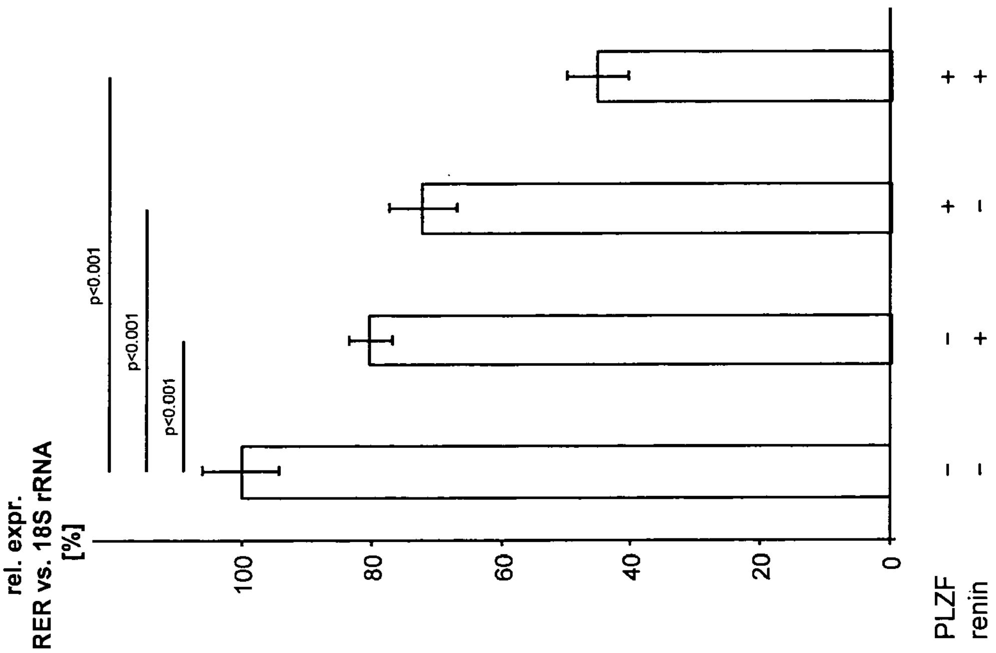
rel. expr.
RER vs. 18S rRNA
[%]
100
80
60
40
20
0
p<0.001
p<0.001
p<0.001
PLZF – – + +
renin – + – +

Fig. 5A

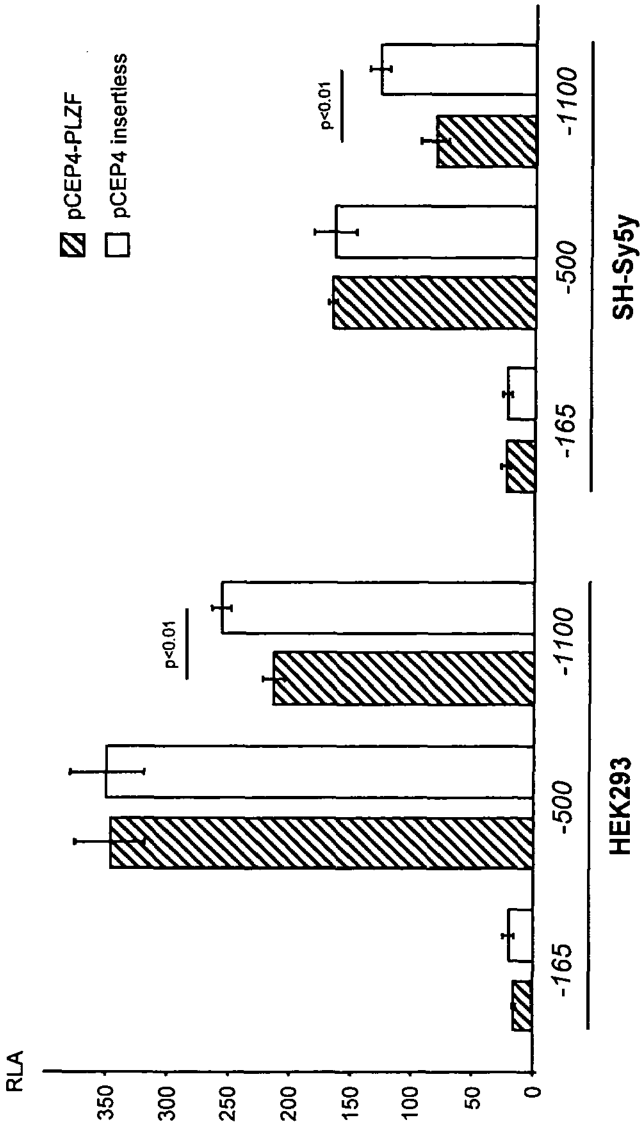

pCEP4-PLZF
pCEP4 insertless
RLA
350
300
250
200
150
100
50
0
p<0.01
p<0.01
-165
-500
-1100
-165
-500
-1100
HEK293
SH-Sy5y


Fig. 5B

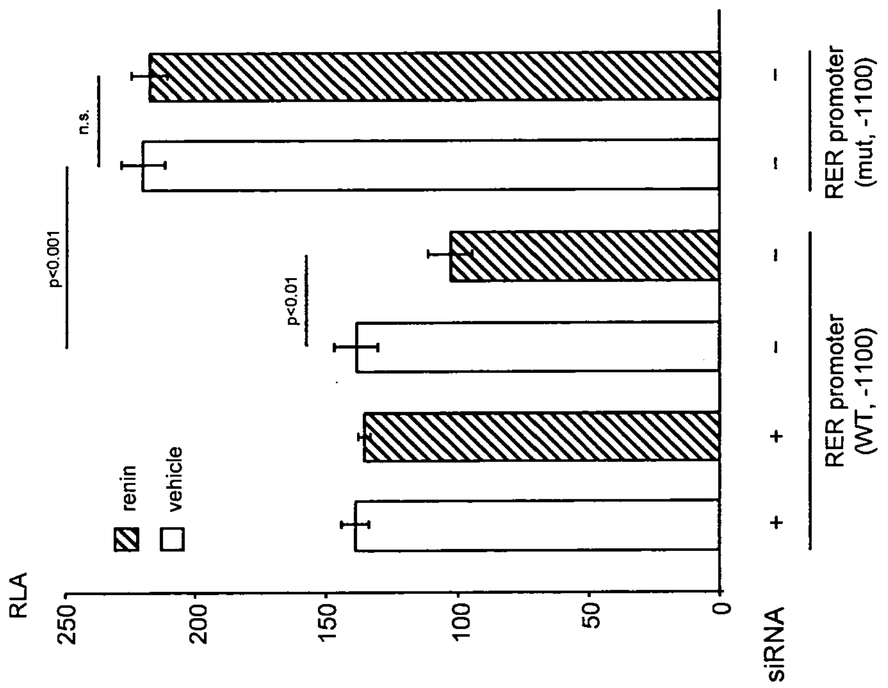
RLA
250
200
150
100
50
0
siRNA
renin
vehicle
p<0.001
p<0.01
n.s.
+
+
–
–
–
–
RER promoter
(WT, -1100)
RER promoter
(mut, -1100)

Fig. 6A

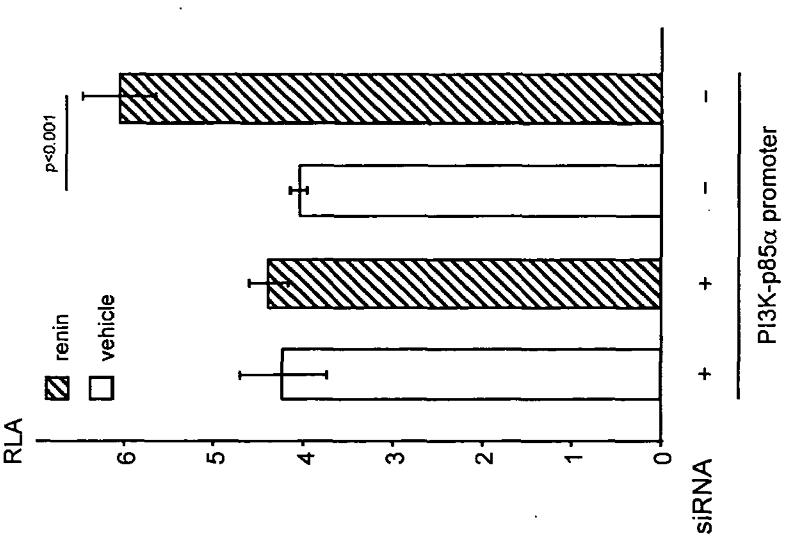


Fig. 6B

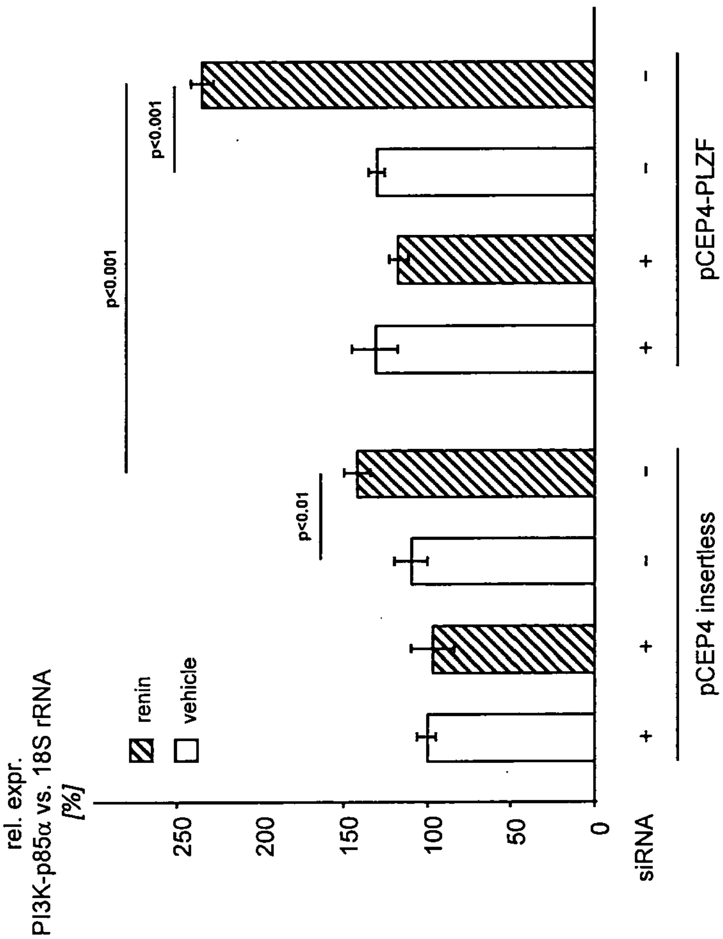
rel. expr.
PI3K-p85α vs. 18S rRNA
[%]
renin
vehicle
250
200
150
100
50
0
siRNA
+
+
-
-
+
+
-
-
p<0.01
p<0.001
p<0.001
pCEP4 insertless
pCEP4-PLZF

Fig. 6C

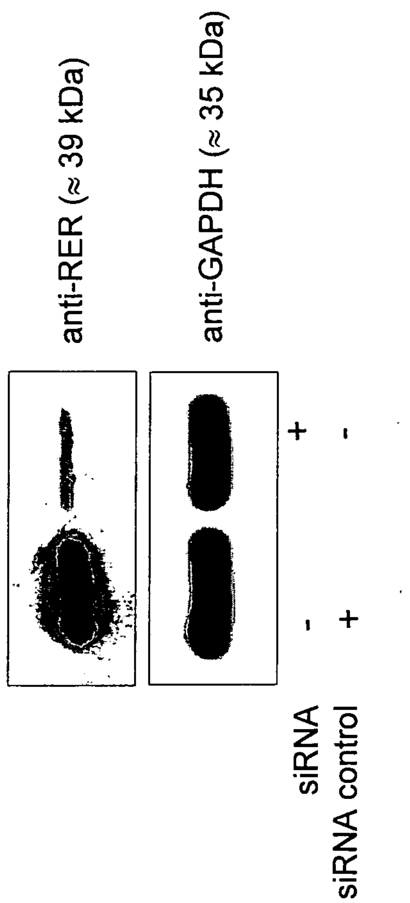


Fig. 6D

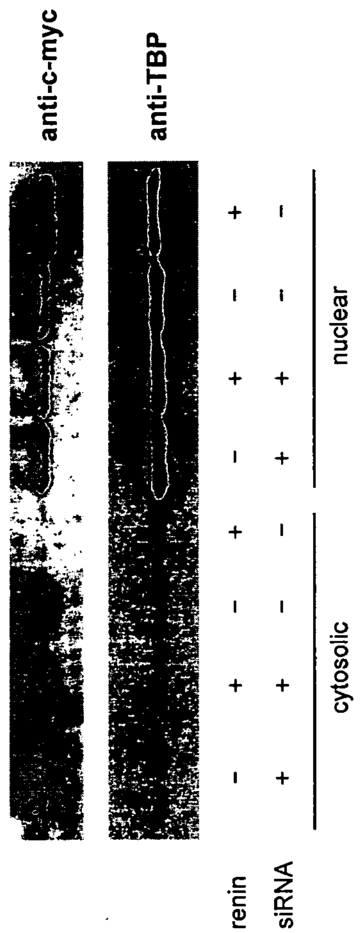


Fig. 7A

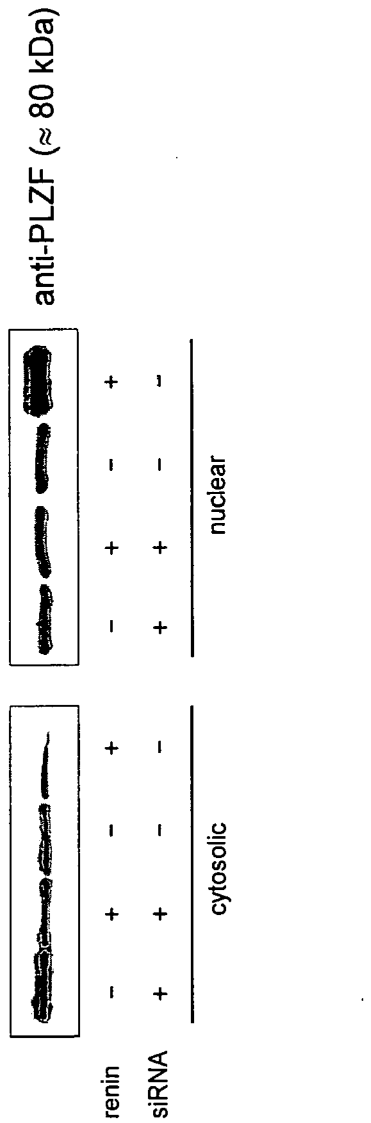


Fig. 7B

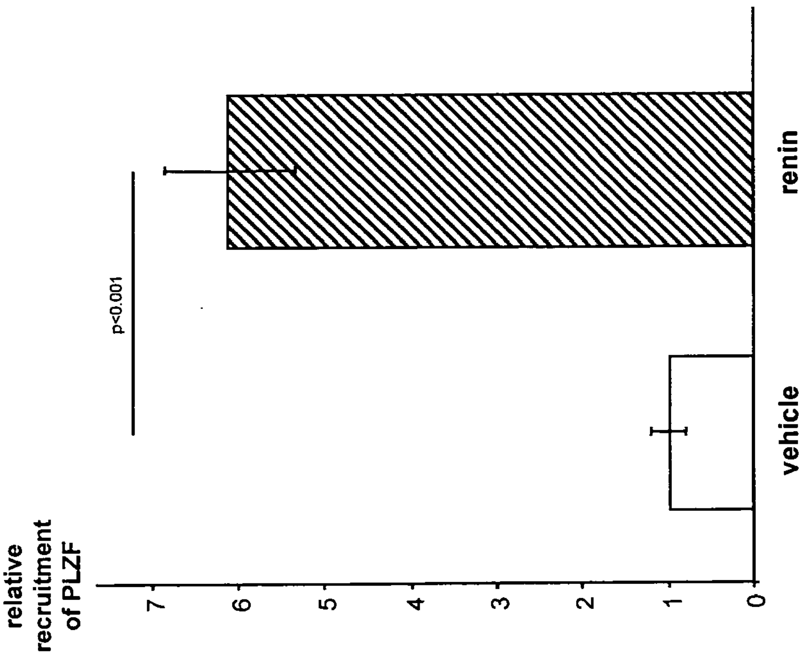
relative recruitment of PLZF
0
1
2
3
4
5
6
7
p<0.001
vehicle
renin

Fig. 7C

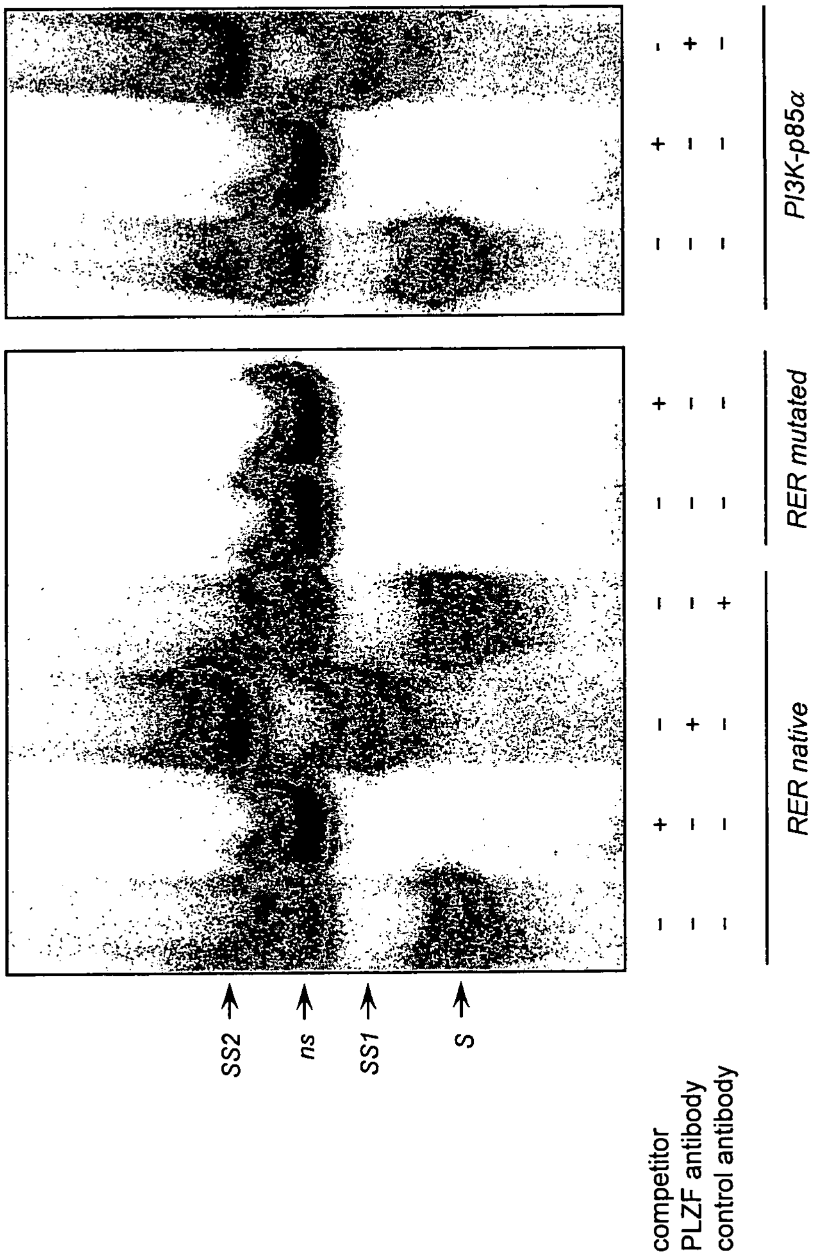
competitor
PLZF antibody
control antibody
SS2
ns
SS1
S
- - -
+ - -
- + -
- - +
- - -
+ - -
RER native
RER mutated
- - -
+ - -
- + -
PI3K-p85α

Fig. 7D

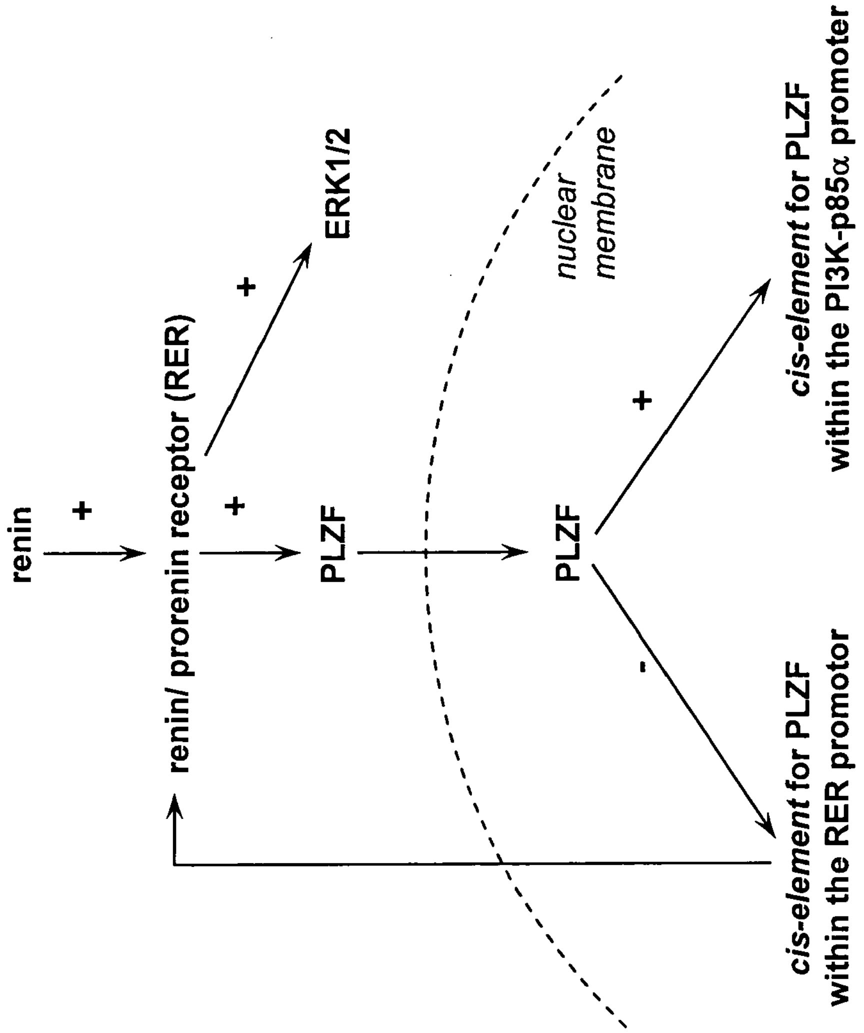
renin
+
renin/ prorenin receptor (RER)
+
ERK1/2
+
PLZF
PLZF
nuclear membrane
+
cis-element for PLZF within the PI3K-p85α promoter
-
cis-element for PLZF within the RER promotor

Fig. 8

European Patent Office

# EUROPEAN SEARCH REPORT

Application Number
EP 06 01 6940

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A<br><br>X | FR 2 815 964 A (INST NAT SANTE RECH MED [FR]) 3 May 2002 (2002-05-03)<br>* claims 3,6; sequences 5, 6 *<br>* page 16, lines 20-32 *<br>* abstract *<br>* page 5, lines 11,12 *<br>* page 6, line 22 - page 7, line 2 * | 1-19,<br>21-28,30<br>20,29 | INV.<br>G01N33/68<br>C07K14/72<br>G01N33/74<br>C12Q1/34 |
| X | & DATABASE EMBL [Online] 5 June 2002 (2002-06-05), "Homo sapiens renin receptor mRNA, complete cds." retrieved from EBI accession no. EMBL:AF291814 Database accession no. AF291814<br>* the whole document * | 20,29 | |
| X | & DATABASE Geneseq [Online] 9 August 2002 (2002-08-09), "Human renin receptor intracellular domain coding sequence." retrieved from EBI accession no. GSN:ABN83322 Database accession no. ABN83322<br>* the whole document *<br>-----<br>-/-- | 20,29 | TECHNICAL FIELDS SEARCHED (IPC)<br>G01N<br>C07K<br>C12Q |

The present search report has been drawn up for all claims

| Place of search | Date of completion of the search | Examiner |
|---|---|---|
| The Hague | 11 May 2007 | Boiangiu, Clara |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 06 01 6940

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,A | SENBONMATSU TAKAAKI ET AL: "A novel angiotensin II type 2 receptor signaling pathway: possible role in cardiac hypertrophy." THE EMBO JOURNAL 15 DEC 2003, vol. 22, no. 24, 15 December 2003 (2003-12-15), pages 6471-6482, XP002433106 ISSN: 0261-4189 * abstract * * page 6472, right-hand column, paragraph 2 - page 6473, left-hand column, paragraph 3 * * page 6476, left-hand column, paragraph 2 - page 6478, left-hand column, paragraph 1 * ----- | 1-30 | |
| A | LEVY ET AL: "How to Explain the Differences Between Renin Angiotensin System Modulators" AMERICAN JOURNAL OF HYPERTENSION, ELSEVIER, vol. 18, no. 9, September 2005 (2005-09), pages 134-141, XP005041829 ISSN: 0895-7061 * the whole document * ----- | 1-30 | TECHNICAL FIELDS SEARCHED (IPC) |
| A | EP 1 391 462 A1 (OTSUKA PHARMA CO LTD [JP]) 25 February 2004 (2004-02-25) * the whole document * ----- -/-- | 1-30 | |

The present search report has been drawn up for all claims

| Place of search | Date of completion of the search | Examiner |
|---|---|---|
| The Hague | 11 May 2007 | Boiangiu, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

3

EPO FORM 1503 03.82 (P04C01)

European Patent Office

# EUROPEAN SEARCH REPORT

Application Number
EP 06 01 6940

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | NGUYEN G ET AL: "MISE EN EVIDENCE D'UN RECEPTEUR DE LA RENINE SUR LES CELLULES MESANGIALES HUMAINES: EFFETS SUR LE PAI1 SUR LE GMPC" NEPHROLOGIE, EDITIONS MEDECINE ET HYGIENE, GENEVA, CH, vol. 19, no. 7, 1998, pages 411-416, XP001023252 ISSN: 0250-4960 * the whole document * ----- | 1-30 | |
| A | SEALEY J E ET AL: "SPECIFIC PRORENIN / RENIN BINDING (PROBP) IDENTIFICATION AND CHARACTERIZATION OF A NOVEL MEMBRANE SITE" AMERICAN JOURNAL OF HYPERTENSION, NEW YORK, NY, US, vol. 9, no. 5, 1996, pages 491-502, XP001010230 * the whole document * ----- | 1-30 | |
| A | NGUYEN G ET AL: "Pivotal role of the renin/prorenin receptor in angiotensin II production and cellular responses to renin" JOURNAL OF CLINICAL INVESTIGATION, NEW YORK, NY, US, vol. 109, no. 11, June 2002 (2002-06), pages 1417-1427, XP002227437 ISSN: 0021-9738 * abstract; figure 1 * ----- -/-- | 1-30 | TECHNICAL FIELDS SEARCHED (IPC) |

The present search report has been drawn up for all claims

| Place of search | Date of completion of the search | Examiner |
|---|---|---|
| The Hague | 11 May 2007 | Boiangiu, Clara |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 06 01 6940

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| P,X | SCHEFE JAN H ET AL: "A novel signal transduction cascade involving direct physical interaction of the renin/prorenin receptor with the transcription factor promyelocytic zinc finger protein." CIRCULATION RESEARCH 8 DEC 2006, vol. 99, no. 12, 8 December 2006 (2006-12-08), pages 1355-1366, XP008078632 ISSN: 1524-4571 * the whole document * ----- | 1-30 | |
| P,X | DE MELLO WALMOR C: "On the pathophysiological implications of an intracellular renin receptor" CIRCULATION RESEARCH, vol. 99, no. 12, December 2006 (2006-12), pages 1285-1286, XP008078610 ISSN: 0009-7330 * the whole document * ----- | 1-30 | |
| | | | TECHNICAL FIELDS SEARCHED (IPC) |

The present search report has been drawn up for all claims

3

| Place of search | Date of completion of the search | Examiner |
|---|---|---|
| The Hague | 11 May 2007 | Boiangiu, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..................................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 06 01 6940

This annex lists the patent family members relating to the patent documents cited in the above-mentioned European search report.
The members are as contained in the European Patent Office EDP file on
The European Patent Office is in no way liable for these particulars which are merely given for the purpose of information.

11-05-2007

| Patent document cited in search report | | Publication date | Patent family member(s) | | | Publication date |
|---|---|---|---|---|---|---|
| FR 2815964 | A | 03-05-2002 | WO | 0236630 | A2 | 10-05-2002 |
| EP 1391462 | A1 | 25-02-2004 | AU | 2002253564 | A2 | 11-11-2002 |
| | | | CA | 2445309 | A1 | 07-11-2002 |
| | | | CN | 1505637 | A | 16-06-2004 |
| | | | WO | 02088175 | A1 | 07-11-2002 |
| | | | MX | PA03009778 | A | 27-05-2004 |
| | | | US | 2004175763 | A1 | 09-09-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **AMSTERDAM A ; NISSEN RM ; SUN Z ; SWINDELL EC ; FARRINGTON S ; HOPKINS N.** Identification of 315 genes essential for early zebrafish development. *Proc Natl Acad Sci USA,* 2004, vol. 101, 12792-12797
- **AVANTAGGIATO V ; PANDOLFI PP ; RUTHARDT M ; HAWE N ; ACAMPORA D ; PELICCI PG ; SIMEONE A.** Developmental analysis of murine Promyelocyte Leukemia Zinc Finger (PLZF) gene expression: implications for the neuromeric model of the forebrain organization. *J Neurosci,* 1995, vol. 15, 4927-4942
- **BARNA M ; HAWE N ; NISWANDER L ; PANDOLFI PP.** Plzf regulates limb and axial skeletal patterning. *Nat Genet,* 2000, vol. 25, 166-172
- **BARNA M ; MERGHOUB T ; COSTOYA JA ; RUGGERO D ; BRANFORD M ; BERGIA A ; SAMORI B ; PANDOLFI PP.** Plzf mediates transcriptional repression of HoxD gene expression through chromatin remodeling. *Dev Cell,* 2002, vol. 3, 499-510
- **BRIVANLOU AH ; DARNELL JE, JR.** Signal transduction and the control of gene expression. *Science,* 2002, vol. 295, 813-818
- **BRYANT GO ; PTASHNE M.** Independent recruitment in vivo by Gal4 of two complexes required for transcription. *Mol Cell,* 2003, vol. 11, 1301-1309
- **BURCKLE CA ; JAN DANSER AH ; MULLER DN ; GARRELDS IM ; GASC JM ; POPOVA E ; PLEHM R ; PETERS J ; BADER M ; NGUYEN G.** Elevated blood pressure and heart rate in human renin receptor transgenic rats. *Hypertension,* 2006, vol. 47, 552-556
- **CARTHARIUS K ; FRECH K ; GROTE K ; KLOCKE B ; HALTMEIER M ; KLINGENHOFF A ; FRISCH M ; BAYERLEIN M ; WERNER T.** MatInspector and beyond: promoter analysis based on transcription factor binding sites. *Bioinformatics,* 2005, vol. 21, 2933-2942
- **CLAUSMEYER S ; STURZEBECHER R ; PETERS J.** An alternative transcript of the rat renin gene can result in a truncated prorenin that is transported into adrenal mitochondria. *Circ Res,* 1999, vol. 84, 337-344
- **COSTOYA JA ; PANDOLFI PP.** The role of promyelocytic leukemia zinc finger and promyelocytic leukemia in leukemogenesis and development. *Curr Opin Hematol,* 2001, vol. 8, 212-217
- **DANSER AH ; DEINUM J.** Renin, prorenin and the putative (pro)renin receptor. *Hypertension,* 2005, vol. 46, 1069-1076
- **EMERY JG ; OHLSTEIN EH ; JAYE M.** Therapeutic modulation of transcription factor activity. *Trends Pharmacol Sci,* 2001, vol. 22, 233-240
- **FUNKE-KAISER H ; BOLBRINKER J ; THEIS S ; LEMMER J ; RICHTER CM ; PAUL M ; ORZECHOWSKI HD.** Characterization of the c-specific promoter of the gene encoding human endothelin-converting enzyme-1 (ECE-1). *FEBS Lett,* 2000, vol. 466, 310-316
- **FUNKE-KAISER H ; LEMMER J ; LANGSDORFF CV ; THOMAS A ; KOVACEVIC SD ; STRASDAT M ; BEHROUZI T ; ZOLLMANN FS ; PAUL M ; ORZECHOWSKI HD.** Endothelin-converting enzyme-1 (ECE-1) is a downstream target of the homeobox transcription factor Nkx2-5. *Faseb J,* 2003, vol. 17, 1487-1489
- **FUNKE-KAISER H ; REICHENBERGER F ; KOPKE K ; HERRMANN SM ; PFEIFER J ; ORZECHOWSKI HD ; ZI- DEK W ; PAUL M ; BRAND E.** Differential binding of transcription factor E2F-2 to the endothelin-converting enzyme-1b promoter affects blood pressure regulation. *Hum Mol Genet,* 2003, vol. 12, 423-433
- **FUNKE-KAISER H ; THOMAS A ; BREMER J ; KOVACEVIC SD ; SCHEUCH K ; BOLBRINKER J ; THEIS S ; LEMMER J ; ZIMMERMANN A ; ZOLLMANN FS.** Regulation of the major isoform of human endothelin-converting enzyme-1 by a strong housekeeping promoter modulated by polymorphic microsatellites. *J Hypertens,* 2003, vol. 21, 2111-2124
- **GAYNOR EC ; HEESEN S ; GRAHAM TR ; AEBI M ; EMR SD.** Signal-mediated retrieval of a membrane protein from the Golgi to the ER in yeast. *J Cell Biol,* 1994, vol. 127, 653-665
- **GLASER KB ; STAVER MJ ; WARING JF ; STENDER J ; ULRICH RG ; DAVIDSEN SK.** Gene expression profiling of multiple histone deacetylase (HDAC) inhibitors: defining a common gene set produced by HDAC inhibition in T24 and MDA carcinoma cell lines. *Mol Cancer Ther,* 2003, vol. 2, 151-163

- **GRIGNANI F ; DE MATTEIS S ; NERVI C ; TOMASSONI L ; GELMETTI V ; CIOCE M ; FANELLI M ; RUTHARDT M ; FERRARA FF ; ZAMIR I.** Fusion proteins of the retinoic acid receptor-alpha recruit histone deacetylase in promyelocytic leukaemia. *Nature,* 1998, vol. 391, 815-818
- **HERSHEY JC ; STEINER, B ; FISCHLI, W. ; FEUERSTEIN, G.** Renin inhibitors: An antihypertensive strategy on the verge of reality. *Drug Discovery Today: Therapeutic Strategies,* 2005, vol. 2, 181-185
- **ICHIHARA A ; HAYASHI M ; KANESHIRO Y ; SUZUKI F ; NAKAGAWA T ; TADA Y ; KOURA Y ; NISHIYAMA A ; OKADA H ; UDDIN MN.** Inhibition of diabetic nephropathy by a decoy peptide corresponding to the "handle" region for nonproteolytic activation of prorenin. *J Clin Invest,* 2004, vol. 114, 1128-1135
- **ICHIHARA A ; KANESHIRO, Y ; TAKEMITSU, T ; SUZUKI, F ; NAKAGAWA, T ; NISHIYAMA, A ; IWATA, H ; ISHIDA, Y ; INAGAMI, T ; SARUTA, T.** Receptor-associated prorenin system" contributes to hypertensive end-organ damage. *J Hypertens,* 2005, vol. 23 (2), 78
- **IVINS S ; PEMBERTON K ; GUIDEZ F ; HOWELL L ; KRUMLAUF R ; ZELENT A.** Regulation of Hoxb2 by APL-associated PLZF protein. *Oncogene,* 2003, vol. 22, 3685-3697
- **JUILLERAT-JEANNERET L ; CELERIER J ; CHAPUIS BERNASCONI C ; NGUYEN G ; WOSTL W ; MAERKI HP ; JANZER RC ; CORVOL P ; GASC JM.** Renin and angiotensinogen expression and functions in growth and apoptosis of human glioblastoma. *Br J Cancer,* 2004, vol. 90, 1059-1068
- **LABBAYE C ; QUARANTA MT ; PAGLIUCA A ; MILITI S ; LICHT JD ; TESTA U ; PESCHLE C.** PLZF induces megakaryocytic development, activates Tpo receptor expression and interacts with GATA1 protein. *Oncogene,* 2002, vol. 21, 6669-6679
- **LEE-KIRSCH MA ; GAUDET F ; CARDOSO MC ; LINDPAINTNER K.** Distinct renin isoforms generated by tissue-specific transcription initiation and alternative splicing. *Circ Res,* 1999, vol. 84, 240-246
- **LI JY ; ENGLISH MA ; BALL HJ ; YEYATI PL ; WAXMAN S ; LICHT JD.** Sequence-specific DNA binding and transcriptional regulation by the promyelocytic leukemia zinc finger protein. *JBiol Chem,* 1997, vol. 272, 22447-22455
- **LUDWIG J ; KERSCHER S ; BRANDT U ; PFEIFFER K ; GETLAWI F ; APPS DK ; SCHAGGER H.** Identification and characterization of a novel 9.2-kDa membrane sector-associated protein of vacuolar proton-ATPase from chromaffin granules. *J Biol Chem,* 1998, vol. 273, 10939-10947
- **MAZAK I ; WELLNER, M ; SHAGDARSUREN, E ; DECHEND, R ; SCHULZE-FORSTER, K ; NGUYEN, G ; LUFT, F.C ; MULLER, D.N.** Renin induces ERK 1/2 phosphorylation in U937 monocyte/macrophages independent of angiotensin II. *Abstract book, 57th annual fall conference and scientific sessions of the CHBPR,* 2003
- **MINUCCI S ; PELICCI PG.** Histone deacetylase inhibitors and the promise of epigenetic (and more) treatments for cancer. *Nat Rev Cancer,* 2006, vol. 6, 38-51
- **MULLER L ; BARRET A ; ETIENNE E ; MEIDAN R ; VALDENAIRE O ; CORVOL P ; TOUGARD C.** Het-erodimerization of endothelin-converting enzyme-1 isoforms regulates the subcellular distribution of this metalloprotease. *J Biol Chem,* 2003, vol. 278, 545-555
- **NELSON N ; HARVEY WR.** Vacuolar and plasma membrane proton-adenosinetriphosphatases. *Physiol Rev,* 1999, vol. 79, 361-385
- **NGUYEN G ; BURCKLE CA ; SRAER JD.** Renin/prorenin-receptor biochemistry and functional significance. *Curr Hypertens Rep,* 2004, vol. 6, 129-132
- **NGUYEN G ; DELARUE F ; BERROU J ; RONDEAU E ; SRAER JD.** Specific receptor binding of renin on human mesangial cells in culture increases plasminogen activator inhibitor-1 antigen. *Kidney Int,* 1996, vol. 50, 1897-1903
- **NGUYEN G ; DELARUE F ; BURCKLE C ; BOUZHIR L ; GILLER T ; SRAER JD.** Pivotal role of the renin/prorenin receptor in angiotensin II production and cellular responses to renin. *J Clin Invest,* 2002, vol. 109, 1417-1427
- **NUSSBERGER J ; WUERZNER G ; JENSEN C ; BRUNNER HR.** Angiotensin II suppression in humans by the orally active renin inhibitor Aliskiren (SPP100): comparison with enalapril. *Hypertension,* 2002, vol. 39, E1-8
- **ORZECHOWSKI HD ; GUNTHER A ; MENZEL S ; ZIMMERMANN A ; FUNKE-KAISER H ; REAL R ; SUBKOWSKI T ; ZOLLMANN FS ; PAUL M.** Transcriptional mechanism of protein kinase C-induced isoform-specific expression of the gene for endothelin-converting enzyme-1 in human endothelial cells. *Mol Pharmacol,* 2001, vol. 60, 1332-1342
- **PETERS J ; CLAUSMEYER S.** Intracellular sorting of renin: cell type specific differences and their consequences. *J Mol Cell Cardiol,* 2002, vol. 34, 1561-1568
- **RAMAKERS C ; RUIJTER JM ; DEPREZ RH ; MOORMAN AF.** Assumption-free analysis of quantitative real-time polymerase chain reaction (PCR) data. *Neurosci Lett,* 2003, vol. 339, 62-66

- **RAMSER J ; ABIDI FE ; BURCKLE CA ; LENSKI C ; TORIELLO H ; WEN G ; LUBS HA ; ENGERT S ; STEVENSON RE ; MEINDL A.** A unique exonic splice enhancer mutation in a family with X-linked mental retardation and epilepsy points to a novel role of the renin receptor. *Hum Mol Genet,* 2005, vol. 14, 1019-1027
- **RE RN.** Intracellular renin and the nature of intracrine enzymes. *Hypertension,* 2003, vol. 42, 117-122
- **SENBONMATSU T ; SAITO T ; LANDON EJ ; WATANABE O ; PRICE E, JR ; ROBERTS RL ; IMBODEN H ; FITZGERALD TG ; GAFFNEY FA ; INAGAMI T.** A novel angiotensin II type 2 receptor signaling pathway: possible role in cardiac hypertrophy. *Embo J,* 2003, vol. 22, 6471-6482
- **SINN PL ; SIGMUND CD.** Identification of three human renin mRNA isoforms from alternative tissue-specific transcriptional initiation. *Physiol Genomics,* 2000, vol. 3, 25-31
- **VINCENT MJ ; MARTIN AS ; COMPANS RW.** Function of the KKXX motif in endoplasmic reticulum retrieval of a transmembrane protein depends on the length and structure of the cytoplasmic domain. *J Biol Chem,* 1998, vol. 273, 950-956
- **WANG J ; KIRBY CE ; HERBST R.** The tyrosine phosphatase PRL-1 localizes to the endoplasmic reticulum and the mitotic spindle and is required for normal mitosis. *J Biol Chem,* 2002, vol. 277, 46659-46668
- **YEYATI PL ; SHAKNOVICH R ; BOTERASHVILI S ; LI J ; BALL HJ ; WAXMAN S ; NASON-BURCHENAL K ; DMITROVSKY E ; ZELENT A ; LICHT JD.** Leukemia translocation protein PLZF inhibits cell growth and expression of cyclin A. *Oncogene,* 1999, vol. 18, 925-934